# EUROPEAN PATENT APPLICATION

(11) **EP 2 987 500 A1**
(43) Date of publication of application: **24.02.2016**
(21) Application number: 15175346.4
(22) Date of filing: 17.09.2010
(51) Int. Cl.: A61K 39/21, C07K 14/035, C07K 19/00, C07K 14/005, A61K 39/12, A61K 39/00

(54) **IMMUNOLOGICAL COMPOSITIONS FOR HIV**

(30) Priority: 17.09.2009 US 243522 P
(62) Divisional of application: 10755306.7
(71) Applicant: Sanofi Pasteur Inc., Swiftwater, PA 18370-9100 (US); The United States of America, as represented by The Secretary of the Army, on behalf of The Walter Reed Army Institute of Rese, Fort Dettrick, MD 21702-5012 (US); Global Solutions For Infectious Diseases, South San Francisco, CA 94080 (US); Ministry Of Public Health, Nonthaburi 11000 (TH)
(72) Inventor: TARTAGLIA, James T., Swiftwater, PA 18370-9100 (US); GURUNATHAN, Sanjay, Swiftwater, PA 18370-9100 (US); KIM, Jerome H., Frederick, MD 21702-5012 (US); FRANCIS, Don, South San Francisco, CA 94080 (US); RERKS-NGARM, Supachai, Nonthaburi, 11000 (TH)
(74) Representative: Cole, William Gwyn

(57) **Abstract**

The disclosure relates to immunological compositions for vaccinating human beings against infection by the Human Immunodeficiency Virus (HIV).

SEQUENCE LISTING

<110> Sanofi Pasteur Ltd.

<120> Immunolgoical Compositions

<130> APL-09-03-PR

<160> 6

<170> PatentIn version 3.5

<210> 1

<211> 1478

<212> DNA

<213> Human immunodeficiency virus

<400> 1

ggtacctgtg tggaaagaag caaccaccac tctattttgt gcatcagatg ctaaagcata 60

tgatacagag gcacataatg tttgggccac acatgcctgt gtacccacag accccaaccc 120

acaagaagta gaattggtaa atgtgacaga aaattttaac atgtggaaaa ataacatggt 180

agaacagatg catgaggata taatcagttt atgggatcaa agcctaaagc catgtgtaaa 240

attaacccca ctctgtgtta ctttaaattg cactgatttg aggaatacta ctaataccaa 300

taatagtact gataataaca atagtaaaag cgagggaaca ataaagggag gagaaatgaa 360

aaactgctct ttcaatatca ccacaagcat aggagataag atgcagaaag aatatgcact 420

tctttataaa cttgatatag aaccaataga taatgatagt accagctata ggttgataag 480

ttgtaatacc tcagtcatta cacaagcttg tccaaagata tcctttgagc caattcccat 540

acactattgt gccccggctg gttttgcgat tctaaagtgt aacgataaaa agttcagtgg 600

aaaaggatca tgtaaaaatg tcagcacagt acaatgtaca catggaatta ggccagtagt 660

atcaactcaa ctgctgttaa atggcagtct agcagaagaa gaggtagtaa ttagatctga 720

ggatttcact gataatgcta aaaccatcat agtacatctg aaagaatctg tacaaattaa 780

ttgtacaaga cccaactaca ataaaagaaa aaggatacat ataggaccag ggagagcatt 840

ttatacaaca aaaaatataa aaggaactat aagacaagca cattgtatca ttagtagagc 900

aaaatggaat gacactttaa gacagatagt tagcaagtta aaagaacaat ttaagaataa 960

aacaatagtc tttaatccat cctcaggagg ggacccagaa attgtaatgc acagttttaa 1020

ttgtggaggg gaatttttct actgtaatac atcaccactg tttaatagta tttggaatgg 1080

taataatact tggaataata ctacagggtc aaataacaat atcacacttc aatgcaaaat 1140

aaaacaaatt ataaacatgt ggcagaaagt aggaaaagca atgtatgccc ctcccattga 1200

aggacaaatt agatgttcat caaatattac agggctacta ttaacaagag atggtggtga 1260

ggacacggac acgaacgaca ccgagatctt cagacctgga ggaggagata tgagggacaa 1320

ttggagaagt gaattatata aatataaagt agtaacaatt gaaccattag gagtagcacc 1380

caccaaggca aagagaagag tggtgcagag agaaaaaaga gcagcgatag gagctctgtt 1440

ccttgggttc ttaggagcag caggaagcac tatgggcg 1478

<210> 2

<211>  492
<212>  PRT
<213>  Human immunodeficiency virus
<400>  2
Val Pro Val Trp Lys Glu Ala Thr Thr Thr Leu Phe Cys Ala Ser Asp
1               5                   10                  15
Ala Lys Ala Tyr Asp Thr Glu Ala His Asn Val Trp Ala Thr His Ala
            20                  25                  30
Cys Val Pro Thr Asp Pro Asn Pro Gln Glu Val Glu Leu Val Asn Val
        35                  40                  45
Thr Glu Asn Phe Asn Met Trp Lys Asn Asn Met Val Glu Gln Met His
    50                  55                  60
Glu Asp Ile Ile Ser Leu Trp Asp Gln Ser Leu Lys Pro Cys Val Lys
65                  70                  75                  80
Leu Thr Pro Leu Cys Val Thr Leu Asn Cys Thr Asp Leu Arg Asn Thr
                85                  90                  95
Thr Asn Thr Asn Asn Ser Thr Asp Asn Asn Asn Ser Lys Ser Glu Gly
            100                 105                 110
Thr Ile Lys Gly Gly Glu Met Lys Asn Cys Ser Phe Asn Ile Thr Thr
        115                 120                 125
Ser Ile Gly Asp Lys Met Gln Lys Glu Tyr Ala Leu Leu Tyr Lys Leu
    130                 135                 140
Asp Ile Glu Pro Ile Asp Asn Asp Ser Thr Ser Tyr Arg Leu Ile Ser
145                 150                 155                 160
Cys Asn Thr Ser Val Ile Thr Gln Ala Cys Pro Lys Ile Ser Phe Glu
                165                 170                 175
Pro Ile Pro Ile His Tyr Cys Ala Pro Ala Gly Phe Ala Ile Leu Lys
            180                 185                 190
Cys Asn Asp Lys Lys Phe Ser Gly Lys Gly Ser Cys Lys Asn Val Ser
        195                 200                 205
Thr Val Gln Cys Thr His Gly Ile Arg Pro Val Val Ser Thr Gln Leu
    210                 215                 220
Leu Leu Asn Gly Ser Leu Ala Glu Glu Glu Val Val Ile Arg Ser Glu
225                 230                 235                 240
Asp Phe Thr Asp Asn Ala Lys Thr Ile Ile Val His Leu Lys Glu Ser
                245                 250                 255
Val Gln Ile Asn Cys Thr Arg Pro Asn Tyr Asn Lys Arg Lys Arg Ile
            260                 265                 270

His Ile Gly Pro Gly Arg Ala Phe Tyr Thr Thr Lys Asn Ile Lys Gly
        275                 280                 285
Thr Ile Arg Gln Ala His Cys Ile Ile Ser Arg Ala Lys Trp Asn Asp
    290                 295                 300
Thr Leu Arg Gln Ile Val Ser Lys Leu Lys Glu Gln Phe Lys Asn Lys
305                 310                 315                 320
Thr Ile Val Phe Asn Pro Ser Ser Gly Gly Asp Pro Glu Ile Val Met
                325                 330                 335
His Ser Phe Asn Cys Gly Gly Glu Phe Phe Tyr Cys Asn Thr Ser Pro
            340                 345                 350
Leu Phe Asn Ser Ile Trp Asn Gly Asn Asn Thr Trp Asn Asn Thr Thr
        355                 360                 365
Gly Ser Asn Asn Asn Ile Thr Leu Gln Cys Lys Ile Lys Gln Ile Ile
    370                 375                 380
Asn Met Trp Gln Lys Val Gly Lys Ala Met Tyr Ala Pro Pro Ile Glu
385                 390                 395                 400
Gly Gln Ile Arg Cys Ser Ser Asn Ile Thr Gly Leu Leu Leu Thr Arg
                405                 410                 415
Asp Gly Gly Glu Asp Thr Asp Thr Asn Asp Thr Glu Ile Phe Arg Pro
            420                 425                 430
Gly Gly Gly Asp Met Arg Asp Asn Trp Arg Ser Glu Leu Tyr Lys Tyr
        435                 440                 445
Lys Val Val Thr Ile Glu Pro Leu Gly Val Ala Pro Thr Lys Ala Lys
    450                 455                 460
Arg Arg Val Val Gln Arg Glu Lys Arg Ala Ala Ile Gly Ala Leu Phe
465                 470                 475                 480
Leu Gly Phe Leu Gly Ala Ala Gly Ser Thr Met Gly
                485                 490

<210> 3
<211> 472
<212> PRT
<213> Human immunodeficiency virus
<400> 3
Val Pro Val Trp Lys Glu Ala Asp Thr Thr Leu Phe Cys Ala Ser Asp
1               5                   10                  15
Ala Lys Ala His Glu Thr Glu Val His Asn Val Trp Ala Thr His Ala
            20                  25                  30

Cys Val Pro Thr Asp Pro Asn Pro Gln Glu Ile Asp Leu Glu Asn Val
        35                  40                  45
Thr Glu Asn Phe Asn Met Trp Lys Asn Asn Met Val Glu Gln Met Gln
    50                  55                  60
Glu Asp Val Ile Ser Leu Trp Asp Gln Ser Leu Lys Pro Cys Val Lys
65                  70                  75                  80
Leu Thr Pro Pro Cys Val Thr Leu His Cys Thr Asn Ala Asn Leu Thr
                85                  90                  95
Lys Ala Asn Leu Thr Asn Val Asn Asn Arg Thr Asn Val Ser Asn Ile
            100                 105                 110
Ile Gly Asn Ile Thr Asp Glu Val Arg Asn Cys Ser Phe Asn Met Thr
        115                 120                 125
Thr Glu Leu Arg Asp Lys Lys Gln Lys Val His Ala Leu Phe Tyr Lys
    130                 135                 140
Leu Asp Ile Val Pro Ile Glu Asp Asn Asn Asp Ser Ser Glu Tyr Arg
145                 150                 155                 160
Leu Ile Asn Cys Asn Thr Ser Val Ile Lys Gln Pro Cys Pro Lys Ile
                165                 170                 175
Ser Phe Asp Pro Ile Pro Ile His Tyr Cys Thr Pro Ala Gly Tyr Ala
            180                 185                 190
Ile Leu Lys Cys Asn Asp Lys Asn Phe Asn Gly Thr Gly Pro Cys Lys
        195                 200                 205
Asn Val Ser Ser Val Gln Cys Thr His Gly Ile Lys Pro Val Val Ser
    210                 215                 220
Thr Gln Leu Leu Leu Asn Gly Ser Leu Ala Glu Glu Glu Ile Ile Ile
225                 230                 235                 240
Arg Ser Glu Asn Leu Thr Asn Asn Ala Lys Thr Ile Ile Val His Leu
                245                 250                 255
Asn Lys Ser Val Val Ile Asn Cys Thr Arg Pro Ser Asn Asn Thr Arg
            260                 265                 270
Thr Ser Ile Thr Ile Gly Pro Gly Gln Val Phe Tyr Arg Thr Gly Asp
        275                 280                 285
Ile Ile Gly Asp Ile Arg Lys Ala Tyr Cys Glu Ile Asn Gly Thr Glu
    290                 295                 300
Trp Asn Lys Ala Leu Lys Gln Val Thr Glu Lys Leu Lys Glu His Phe
305                 310                 315                 320
Asn Asn Lys Pro Ile Ile Phe Gln Pro Pro Ser Gly Gly Asp Leu Glu
                325                 330                 335

Ile Thr Met His His Phe Asn Cys Arg Gly Glu Phe Phe Tyr Cys Asn
            340                 345                 350
Thr Thr Arg Leu Phe Asn Asn Thr Cys Ile Ala Asn Gly Thr Ile Glu
        355                 360                 365
Gly Cys Asn Gly Asn Ile Thr Leu Pro Cys Lys Ile Lys Gln Ile Ile
    370                 375                 380
Asn Met Trp Gln Gly Ala Gly Gln Ala Met Tyr Ala Pro Pro Ile Ser
385                 390                 395                 400
Gly Thr Ile Asn Cys Val Ser Asn Ile Thr Gly Ile Leu Leu Thr Arg
                405                 410                 415
Asp Gly Gly Ala Thr Asn Asn Thr Asn Asn Glu Thr Phe Arg Pro Gly
            420                 425                 430
Gly Gly Asn Ile Lys Asp Asn Trp Arg Asn Glu Leu Tyr Lys Tyr Lys
        435                 440                 445
Val Val Gln Ile Glu Pro Leu Gly Val Ala Pro Thr Arg Ala Lys Arg
    450                 455                 460
Arg Val Val Glu Arg Glu Lys Arg
465                 470

<210> 4
<211> 472
<212> PRT
<213> Human immunodeficiency virus
<400> 4
Val Pro Val Trp Arg Asp Ala Asp Thr Thr Leu Phe Cys Ala Ser Asp
1               5                   10                  15
Ala Lys Ala His Glu Thr Glu Val His Asn Val Trp Ala Thr His Ala
            20                  25                  30
Cys Val Pro Thr Asp Pro Asn Pro Gln Glu Ile Asp Leu Glu Asn Val
        35                  40                  45
Thr Glu Asn Phe Asn Met Trp Lys Asn Asn Met Val Glu Gln Met Gln
    50                  55                  60
Glu Asp Val Ile Ser Leu Trp Asp Gln Ser Leu Lys Pro Cys Val Lys
65                  70                  75                  80
Leu Thr Pro Leu Cys Val Thr Leu His Cys Thr Asn Ala Asn Leu Thr
                85                  90                  95
Lys Ala Asn Leu Thr Asn Val Asn Asn Arg Thr Asn Val Ser Asn Ile
            100                 105                 110

Ile Gly Asn Ile Thr Asp Glu Val Arg Asn Cys Ser Phe Asn Met Thr
        115                 120                 125
Thr Glu Leu Arg Asp Lys Lys Gln Lys Val His Ala Leu Phe Tyr Lys
    130                 135                 140
Leu Asp Ile Val Pro Ile Glu Asp Asn Asn Asp Asn Ser Lys Tyr Arg
145                 150                 155                 160
Leu Ile Asn Cys Asn Thr Ser Val Ile Lys Gln Ala Cys Pro Lys Ile
                165                 170                 175
Ser Phe Asp Pro Ile Pro Ile His Tyr Cys Thr Pro Ala Gly Tyr Ala
            180                 185                 190
Ile Leu Lys Cys Asn Asp Lys Asn Phe Asn Gly Thr Gly Pro Cys Lys
        195                 200                 205
Asn Val Ser Ser Val Gln Cys Thr His Gly Ile Lys Pro Val Val Ser
    210                 215                 220
Thr Gln Leu Leu Leu Asn Gly Ser Leu Ala Glu Glu Glu Ile Ile Ile
225                 230                 235                 240
Arg Ser Glu Asp Leu Thr Asn Asn Ala Lys Thr Ile Ile Val His Leu
                245                 250                 255
Asn Lys Ser Val Val Ile Asn Cys Thr Arg Pro Ser Asn Asn Thr Arg
            260                 265                 270
Thr Ser Ile Thr Ile Gly Pro Gly Gln Val Phe Tyr Arg Thr Gly Asp
        275                 280                 285
Ile Ile Gly Asp Ile Arg Lys Ala Tyr Cys Glu Ile Asn Gly Thr Glu
    290                 295                 300
Trp Asn Lys Ala Leu Lys Gln Val Thr Glu Lys Leu Lys Glu His Phe
305                 310                 315                 320
Asn Asn Lys Pro Ile Ile Phe Gln Pro Pro Ser Gly Gly Asp Leu Glu
                325                 330                 335
Ile Thr Met His His Phe Asn Cys Arg Gly Glu Phe Phe Tyr Cys Asn
            340                 345                 350
Thr Thr Arg Leu Phe Asn Asn Thr Cys Ile Ala Asn Gly Thr Ile Glu
        355                 360                 365
Gly Cys Asn Gly Asn Ile Thr Leu Pro Cys Lys Ile Lys Gln Ile Ile
    370                 375                 380
Asn Met Trp Gln Gly Ala Gly Gln Ala Met Tyr Ala Pro Pro Ile Ser
385                 390                 395                 400
Gly Thr Ile Asn Cys Val Ser Asn Ile Thr Gly Ile Leu Leu Thr Arg
                405                 410                 415

Asp Gly Gly Ala Thr Asn Asn Thr Asn Asn Glu Thr Phe Arg Pro Gly
            420                 425                 430
Gly Gly Asn Ile Lys Asp Asn Trp Arg Asn Glu Leu Tyr Lys Tyr Lys
        435                 440                 445
Val Val Gln Ile Glu Pro Leu Gly Ala Ala Pro Thr Arg Ala Lys Arg
    450                 455                 460
Arg Val Val Glu Arg Glu Lys Arg
465                 470

<210> 5
<211> 3796
<212> DNA
<213> Artificial Sequence
<220>
<223> Human immunodeficiency virus
<400> 5
   agtacaataa aaagtattaa ataaaaatac ttacttacga aaaaatgact aattagctat 60
   aaaaacccgg ggttatccct gcctaactct attcactaca gagagtacag caaaaactat 120
   tcttaaacct accaagcctc ctactatcat tatgaataat cttttttctc tctccaccac 180
   tcttcttttg gcgcgggtgg gtgctattcc tagtggttca atttgtacta ctttatattt 240
   atataattca cttctccaat tgtcctttat atttcctcct ccaggtctga aggtctcgtt 300
   agtattattg agaccaccat ctcttgtcaa tagtattcct gtaatatttg atacacaatt 360
   aattcttcca ctgatgggag gagcatacat tgcttgtcct gctccctgcc acatgtttat 420
   aatttgcttt atcttgcatg gaagtatgat agtgccatta cacccctcca tggtttcatt 480
   ttccatgcaa gtattattaa acagtcgtgt tgtattgcaa tagaagaatt cccctctaca 540
   attaaaatga tgcattgtaa tttctagatc tcctcctgag ggtggttgaa agattattgt 600
   cttattatta aagtgctctt ttaatttttt agttaccttt tttaaaactt cattccattt 660
   tgttccatta atctcacaat atgcttttct tatatctcct attatgtctc ctgttctgta 720
   gaatacttgt cctggtccta tatttatact tgttcttgta ttgttggagg gtctggtaca 780
   attgatttct acagatttat taaggtgcac tattatggtt ttggcattgt ttgtgagatt 840
   ttcagatctg attattatct cttcttctgc tagactgcca tttaacagca attgagttga 900
   taccacaggc ttaattccat gtgtgcattg tactgagctg acatttttac atggccctgt 960
   cccattgaaa ttcttatcat tacactttaa aatcgcataa ccagctggag tacaataatg 1020
   tataggaatt ggatcaaagg atatctttgg acaagcctgc ttaatgactg aagtattaca 1080
   atttattaac ctatactcac tactactcgt gttatcttca attggtacta tatcaagctt 1140
   gtagaatagt gcatggacct tctgcttctt atctcttagt tctgtggtca tattaaaaga 1200
   acagtttctt acttcatctg ttatatttcc tattatgtta gggacattgg ttatgttctt 1260
   gacattggtc acattagcat tggtacaatt taaagtaacg cagagaggag ttaactttac 1320
   acatggtttt agactttgat cccataaact gattacatcc tcctgcatct gctctaccat 1380
   gttatttttc cacatgttaa aattttctgt tacattttcc aggtgtaatt cttgtgggtt 1440
   ggggtctgtg ggtacacagg catgtgtggc ccagacattg tgcgcttctg tctcttgtgc 1500
   tttggcatct gatgcacaaa atagggtggt atctgcatct ctccatacag gaaccccata 1560
   ataaactgta acccacaagt tgtctgaggc actacaaatt atcaccaacc caaggatcaa 1620
   agtcccccat ttccacaagt ttggccaagt catctgtgtc tccttcactc tcattacgat 1680
   acaaacttaa cggatatcgc gataatgaaa taatttatga ttatttctcg ctttcaattt 1740
   aacacaaccc tcaagaacct ttgtatttat tttcactttt taagtataga ataaagaagc 1800
   tctaattaat taagctacaa atagtttcgt tttcaccttg tctaataact aattaattaa 1860
   cccggatctt gagataaagt gaaaatatat atcattatat tacaaagtac aattatttag 1920
   gtttaatcat gggtgcgaga gcgtcagtat taagcggggg agaattagat cgatgggaaa 1980
   aaattcggtt aaggccaggg ggaaagaaaa aatataaatt aaaacatata gtatgggcaa 2040
   gcagggagct agaacgattc gcagttaatc ctggcctgtt agaaacatca gaaggctgta 2100
   gacaaatact gggacagcta caaccatccc ttcagacagg atcagaagaa cttagatcat 2160
   tatataatac agtagcaacc ctctattgtg tgcatcaaag gatagagata aaagacacca 2220
   aggaagcttt agacaagata gaggaagagc aaaacaaaag taagaaaaaa gcacagcaag 2280
   cagcagctga cacaggacac agcaatcagg tcagccaaaa ttaccctata gtgcagaaca 2340
   tccaggggca aatggtacat caggccatat cacctagaac tttaaatgca tgggtaaaag 2400
   tagtagaaga gaaggctttc agcccagaag tgatacccat gttttcagca ttatcagaag 2460
   gagccacccc acaagattta aacaccatgc taaacacagt ggggggacat caagcagcca 2520
   tgcaaatgtt aaaagagacc atcaatgagg aagctgcaga atgggataga gtgcatccag 2580
   tgcatgcagg gcctattgca ccaggccaga tgagagaacc aaggggaagt gacatagcag 2640
   gaactactag tacccttcag gaacaaatag gatggatgac aaataatcca cctatcccag 2700
   taggagaaat ttataaaaga tggataatcc tgggattaaa taaaatagta agaatgtata 2760
   gccctaccag cattctggac ataagacaag gaccaaaaga accctttaga gactatgtag 2820
   accggttcta taaaactcta agagccgagc aagcttcaca ggaggtaaaa aattggatga 2880
   cagaaacctt gttggtccaa aatgcgaacc cagattgtaa gactatttta aaagcattgg 2940
   gaccagcggc tacactagaa gaaatgatga cagcatgtca gggagtagga ggacccggcc 3000
   ataaggcaag agttttggct gaagcaatga gccaagtaac aaattcagct accataatga 3060
   tgcagagagg caattttagg aaccaaagaa agattgttaa gtgtttcaat tgtggcaaag 3120
   aagggcacac agccagaaat tgcagggccc ctaggaaaaa gggctgttgg aaatgtggaa 3180
   aggaaggaca ccaaatgaaa gattgtactg agagacaggc taatttttta gggaagatct 3240
   ggccttccta caagggaagg ccagggaatt ttcttcagag cagaccagag ccaacagccc 3300
   caccagaaga gagcttcagg tctggggtag agacaacaac tccccctcag aagcaggagc 3360
   cgatagacaa ggaactgtat cctttaactt ccctcagatc actctttggc aacgacccct 3420
   cgtcacaata aagatagggg ggcaactaaa ggaagctcta ttagatacag gagcagatga 3480
   tacagtatta gaagaaatga gtttgccagg aagatggaaa ccaaaaatga tagggggaat 3540
   tggaggtttt atcaaagtaa gacagtatga tcagatactc atagaaatct gtggacataa 3600
   agctataggt acagtattag taggacctac acctgtcaac ataattggaa gaaatctgtt 3660
   gactcagatt ggttgcactt taaattttta acccgggctg cagctcgagg aattcttttt 3720
   attgattaac tagtcaaatg agtatatata attgaaaaag taaaatataa atcatataat 3780
   aatgaaacga aatatc 3796

<210> 6
<211> 3796
<212> DNA
<213> Artificial Sequence
<220>
<223> Human immunodeficiency virus
<400> 6
tcatgttatt tttcataatt tatttttatg aatgaatgct tttttactga ttaatcgata 60
tttttgggcc ccaataggga cggattgaga taagtgatgt ctctcatgtc gtttttgata 120
agaatttgga tggttcggag gatgatagta atacttatta gaaaaaagag agaggtggtg 180
agaagaaaac cgcgcccacc cacgataagg atcaccaagt taaacatgat gaaatataaa 240
tatattaagt gaagaggtta acaggaaata taaaggagga ggtccagact tccagagcaa 300
tcataataac tctggtggta gagaacagtt atcataagga cattataaac tatgtgttaa 360
ttaagaaggt gactaccctc ctcgtatgta acgaacagga cgagggacgg tgtacaaata 420
ttaaacgaaa tagaacgtac cttcatacta tcacggtaat gtggggaggt accaaagtaa 480
aaggtacgtt cataataatt tgtcagcaca acataacgtt atcttcttaa ggggagatgt 540
taattttact acgtaacatt aaagatctag aggaggactc ccaccaactt tctaataaca 600
gaataataat ttcacgagaa aattaaaaaa tcaatggaaa aaattttgaa gtaaggtaaa 660
acaaggtaat tagagtgtta tacgaaaaga atatagagga taatacagag gacaagacat 720
cttatgaaca ggaccaggat ataaatatga acaagaacat aacaacctcc cagaccatgt 780
taactaaaga tgtctaaata attccacgtg ataataccaa aaccgtaaca aacactctaa 840
aagtctagac taataataga gaagaagacg atctgacggt aaattgtcgt taactcaact 900
atggtgtccg aattaaggta cacacgtaac atgactcgac tgtaaaaatg taccgggaca 960
gggtaacttt aagaatagta atgtgaaatt ttagcgtatt ggtcgacctc atgttattac 1020
atatccttaa cctagtttcc tatagaaacc tgttcggacg aattactgac ttcataatgt 1080
taaataattg gatatgagtg atgatgagca caatagaagt taaccatgat atagttcgaa 1140
catcttatca cgtacctgga agacgaagaa tagagaatca agacaccagt ataattttct 1200
tgtcaaagaa tgaagtagac aatataaagg ataatacaat ccctgtaacc aatacaagaa 1260
ctgtaaccag tgtaatcgta accatgttaa atttcattgc gtctctcctc aattgaaatg 1320
tgtaccaaaa tctgaaacta gggtatttga ctaatgtagg aggacgtaga cgagatggta 1380
caataaaaag gtgtacaatt ttaaaagaca atgtaaaagg tccacattaa gaacacccaa 1440

ccccagacac ccatgtgtcc gtacacaccg ggtctgtaac acgcgaagac agagaacacg 1500
aaaccgtaga ctacgtgttt tatcccacca tagacgtaga gaggtatgtc cttggggtat 1560
tatttgacat tgggtgttca acagactccg tgatgtttaa tagtggttgg gttcctagtt 1620
tcagggggta aaggtgttca aaccggttca gtagacacag aggaagtgag agtaatgcta 1680
tgtttgaatt gcctatagcg ctattacttt attaaatact aataaagagc gaaagttaaa 1740
ttgtgttggg agttcttgga aacataaata aaagtgaaaa attcatatct tatttcttcg 1800
agattaatta attcgatgtt tatcaaagca aaagtggaac agattattga ttaattaatt 1860
gggcctagaa ctctatttca cttttatata tagtaatata atgtttcatg ttaataaatc 1920
caaattagta cccacgctct cgcagtcata attcgccccc tcttaatcta gctacccttt 1980
tttaagccaa ttccggtccc cctttctttt ttatatttaa ttttgtatat catacccgtt 2040
cgtccctcga tcttgctaag cgtcaattag gaccggacaa tctttgtagt cttccgacat 2100
ctgtttatga ccctgtcgat gttggtaggg aagtctgtcc tagtcttctt gaatctagta 2160
atatattatg tcatcgttgg gagataacac acgtagtttc ctatctctat tttctgtggt 2220
tccttcgaaa tctgttctat ctccttctcg ttttgttttc attctttttt cgtgtcgttc 2280
gtcgtcgact gtgtcctgtg tcgttagtcc agtcggtttt aatgggatat cacgtcttgt 2340
aggtccccgt ttaccatgta gtccggtata gtggatcttg aaatttacgt acccattttc 2400
atcatcttct cttccgaaag tcgggtcttc actatgggta caaaagtcgt aatagtcttc 2460
ctcggtgggg tgttctaaat ttgtggtacg atttgtgtca ccccccctgta gttcgtcggt 2520
acgtttacaa ttttctctgg tagttactcc ttcgacgtct taccctatct cacgtaggtc 2580
acgtacgtcc cggataacgt ggtccggtct actctcttgg ttccccttca ctgtatcgtc 2640
cttgatgatc atgggaagtc cttgtttatc ctacctactg tttattaggt ggatagggtc 2700
atcctcttta aatattttct acctattagg accctaattt attttatcat tcttacatat 2760
cgggatggtc gtaagacctg tattctgttc ctggttttct tgggaaatct ctgatacatc 2820
tggccaagat attttgagat tctcggctcg ttcgaagtgt cctccatttt ttaacctact 2880
gtctttggaa caaccaggtt ttacgcttgg gtctaacatt ctgataaaat tttcgtaacc 2940
ctggtcgccg atgtgatctt ctttactact gtcgtacagt ccctcatcct cctgggccgg 3000
tattccgttc tcaaaaccga cttcgttact cggttcattg tttaagtcga tggtattact 3060
acgtctctcc gttaaaatcc ttggtttctt tctaacaatt cacaaagtta acaccgtttc 3120
ttcccgtgtg tcggtcttta acgtcccggg gatccttttt cccgacaacc tttacacctt 3180
tccttcctgt ggtttacttt ctaacatgac tctctgtccg attaaaaaat cccttctaga 3240
ccggaaggat gttcccttcc ggtcccttaa aagaagtctc gtctggtctc ggttgtcggg 3300
gtggtcttct ctcgaagtcc agaccccatc tctgttgttg agggggagtc ttcgtcctcg 3360
gctatctgtt ccttgacata ggaaattgaa gggagtctag tgagaaaccg ttgctgggga 3420
gcagtgttat ttctatcccc ccgttgattt ccttcgagat aatctatgtc ctcgtctact 3480
atgtcataat cttctttact caaacggtcc ttctaccttt ggtttttact atccccctta 3540
acctccaaaa tagtttcatt ctgtcatact agtctatgag tatctttaga cacctgtatt 3600
tcgatatcca tgtcataatc atcctggatg tggacagttg tattaacctt ctttagacaa 3660
ctgagtctaa ccaacgtgaa atttaaaaat tgggcccgac gtcgagctcc ttaagaaaaa 3720

taactaattg atcagtttac tcatatatat taactttttc attttatatt tagtatatta 3780
ttactttgct ttatag 3796

<210> 7
<211> 27
<212> DNA
<213> Artificial Sequence
<220>
<223> Herpes simplex virus 1
<400> 7

Asp Tyr Ala Leu Ala Asp Ala Ser Leu Lys Met Ala Asp Pro Asn
                5                   10                  15
Arg Phe Arg Gly Lys Asp Leu Pro Val Leu Asp Glu Leu
                20                  25

## Description

### Related Applications

This application claims priority to U.S. Ser. No. 61/243,522 filed September 17,2009.

### Field of the Invention

The present invention relates generally to the field of immunology and, in particular to methods and compositions for immunizing and generating protection in a host against infection and disease with HIV.

### Background of the Invention

Human immunodeficiency virus (HIV) is a human retrovirus and is the etiological agent of acquired immunodeficiency syndrome (AIDS). Despite the passage of more than 20 years since the discovery of HIV, no effective vaccine has been found to either ameliorate the disease or to prevent infection. By the end of the year 2007, more than 30 million people worldwide were infected with HIV, with more than 20 million of those people living in sub-Saharan Africa (Report on the Global AIDS Epidemic, Joint United Nations Programme on HIV/AIDS (UNAIDS), 2008).

A hallmark of resistance to future vital infection is the generation of 'neutralising antibodies' capable of recognizing the viral pathogen. Another measure is cellular immunity against infected cells. In typical viral infections, generation of neutralizing antibodies and cellular immunity heralds recovery from infection. In HIV-1 infections, however, neutralizing antibodies and cellular immunity appear very early during the infection and have been associated with only a transient decrease in viral burden. In spite of the generation of neutralizing antibodies and cellular immunity, viral replication in HIV-1 infection rebounds and AIDS (acquired immune deficiency syndrome) develops. Thus, in HIV-1 infections, neutralizing antibodies and cellular immunity are not accurate measures of protective immunity. Protective immunity, meaning the vaccinees are protected against new infections by HIV, is a major unaccomplished goal of those skilled in the art.

Several potential vaccines have been tested in humans but found not to be protective. For examples, polypeptide vaccines based on gp120 have been tested (e.g., AIDSVAX^{®} B/B, AIDSVAX^{®} B/E (Vaxgen)) as solo vaccines or together in a prime-boost format, but have not shown protection against HIV infection (McCarthy, M. Lancet. 362(9397):1728 (2003); Nitayaphan, et al. J. Inf. Dis. 190:702-6 (2004); Pitisuttithum, P. 11th Conf. Retr. Opp. Inf. 2004. 115: Abstract 107). Many studies have also been performed using animal models (e.g., monkeys). However, while primate data are instructive they also highlight the gaps in our understanding of immunological mechanism that mediate vaccine associated protection and emphasize the need to conduct human efficacy studies to test promising candidate vaccines empirically.

ALVAC-HIV (vCP1521) vaccine is a preparation of recombinant canarypox-derived virus expressing the products of the HIV-I *env* and *gag* genes. The genes are inserted into the C6 locus under the control of the vaccinia virus H6 and I3L promoters respectively. The gp120 *env* sequence is derived from the HIV-92TH023 (subtype E) strain, but the anchoring part of gp41 is derived from the HIV-LAI (subtype B) strain. ALVAC-HIV infected cells present *env* and *gag* proteins in a near-native conformation (Fang, et al. J. Infect Dis. 180 (4); 1122-32 (1999)). In addition, intracellular processing of the HIV-1 proteins via the MHC class I pathway facilitates stimulation of cytotoxic T-lymphocytes. Part of the rationale for use of Gag from a subtype B in Thailand is that portions of the *gag* gene are conserved among virus subtypes. Therefore, *gag*-specific CTL elicited by vCP1521 may cross-react with CTL epitopes on non-subtype B primary viruses. Data from an AVEG-sponsored prime-boost trial (vCP205 alone or boosted with Chiron SF2 gp120/MF59) showed that CD8* CTL from some vaccine recipients recognized target cells infected with non-subtype B viruses, including subtype E (Ferrari, et al. Proc. Natl. Acad. Sci. USA, 94:1396-401 (1997)).

In view of this data, several attempts have been made to provide protection using a prime-boost immunization format (McNeil, et al. Science. 303:961 (2004)). For example, AIDSVAX^{®} B/E (VaxGen) and ALVAC-HIV have been used as the prime and boost compositions, respectively, and shown to induce neutralizing antibodies (Karnasuta, et al. Vaccine, 23: 2522-2529 (2005). In one safety trial, neutralizing antibodies were observed in 84% to 100% of subjects, cytotoxic lymphocyte (CTL) were observed in 16-25% of subjects, and lymphoproliferation was observed in 55-93% of subjects. In another safety trial, neutralizing antibodies were observed in 31% to 71% of subjects, cytotoxic lymphocytes (CTL) were observed in about 25% of subjects, and lympho-proliferation was observed in 58-71% of subjects. However, protection against infection by HIV was not shown, leading some to question the value of such a combination vaccine (Burton, et al. Science. 303; 316 (2004); Letters to the Editor, Science. 305:177-180 (2004)).

To date, no vaccine has shown protection against HIV infection. Thus, there is a clear need in the art for compositions and methods for protectively immunizing humans against HIV. Such compositions and methods are provided by this disclosure.

### Brief Description of the Drawings

**Figure 1****.** Comparison of the predicted amino acid sequence for A244 rgp120/HIV-1 protein (gd244) with the predicted sequence for MN rgp120/HIV-1 protein (MN.mature).
**Figure 2****.** A. Map of ALVAC-HIV (vCP1521) genome. **B.** Nucleotide sequence of the HIV insert contained within ALVAC-HIV(vCP1521).
**Figure 3****.** Efficacy of the AIDSVAX^{®} B/E ALVAC-HIV prime-boost vaccine.

### Summary of the Invention

The reagents and methodologies described herein may be used to protectively immunize a human being against human immunodeficiency virus, which has not been previously demonstrated. In one embodiment, a two-part composition including a first composition comprising an expression vector encoding an HIV immunogen, and a second composition comprising a polypeptide derived from or representing an HIV immunogen is provided. As shown herein, administration of the first composition prior to the second composition, and then administering the first and second compositions, protects human beings against infection by HIV. In an exemplary embodiment, the first composition includes a live, attenuated viral vector encoding at least one HIV immunogen, and the second composition includes an HIV immunogen in the form of a polypeptide. In certain embodiments, a method for protectively immunizing a human being against human immunodeficiency virus (HIV) by administering to the human being a vaccine composition. The composition consists essentially of a first composition and a second composition, the first composition consisting essentially of a live, attenuated avipox (e.g., canarypox, ALVAC) vector encoding multiple HIV immunogens, and the second composition including one or more polypeptides corresponding in amino acid sequence to at least a portion of HIV gp120. In some embodiments, at least one of the compositions comprises an amino acid sequence corresponding to that of HIV and at least one amino acid sequence corresponding to a herpes simplex virus (HSV). In some embodiments, the first composition is administered to the human being and the first and second compositions are subsequently administered to the human being. In certain embodiments, the compositions are administered via the intramuscular route. Using this method, it has been found for the first time that human beings may be protected from infection by HIV. In certain embodiments, the method involves administering the vaccine to a population of human beings and at least about one-third of that population is protested from infection by HIV.

### Detailed Description

The present invention provides compositions and methodologies useful for treating and / or preventing conditions relating to an infection or other agent(s) such as a tumor cell by stimulating an immune response against such an agent. In general, the immune response results from expression of an immunogen derived from or related to such an agent following administration of a nucleic acid vector encoding the immunogen, for example. In certain embodiments, multiple immunogens (which may be the same or different) are utilized. In other embodiments, variants or derivatives (i.e., by substitution, deletion or addition of amino acids or nucleotides encoding the same) of an immunogen or immunogen (which may be the same or different) may be utilized.

An immunogen may be a moiety (e.g., polypeptide, peptide or nucleic acid) that induces or enhances the immune response of a host to whom or to which the immunogen is administered. An immune response may be induced or enhanced by either increasing or decreasing the frequency, amount, or half-life of a particular immune modulator (e.g, the expression of a cytokine, chemokine, co-stimulatory molecule). This may be directly observed within a host cell containing a polynucleotide of interest (e.g., following infection by a recombinant virus) or within a nearby cell or tissue (e.g., indirectly). The immune response is typically directed against a target antigen. For example, an immune response may result from expression of an immunogen in a host following administration of a nucleic acid vector encoding the immunogen to the host. The immune response may result in one or more of an effect (e.g., maturation, proliferation, direct- or cross-presentation of antigen, gene expression profile) on cells of either the innate or adaptive immune system. For example, the immune response may involve, effect, or be detected in innate immune cells such as, for example, dendritic cells, monocytes, macrophages, natural killer cells, and/or granulocytes (e.g., neutrophils, basophils or eosinophils). The immune response may also involve, effect, or be detected in adaptive immune cells including, for example, lymphocytes (e.g., T cells and / or B cells). The immune response may be observed by detecting such involvement or effects including, for example, the presence, absence, or altered (e.g., increased or decreased) expression or activity of one or more immunomodulators such as a hormone, cytokine, interleukin (e.g., any of IL-1 through IL-35), interferon (e.g., any of IFN-I (IFN-α, IFN-β, IFN-ε, IFN-κ, IFN-τ, IFN-ζ, IFN-ω), IFN-II (e.g., IFN-γ), IFN-III (IFN-λ1, IFN-λ2, IFN-λ3)), chemokine (e.g., any CC cytokine (e.g., any of CCL1 through CCL28), any CXC chemokine (e.g., any of CXCL1 through CXCL24), Mipla), any C chemokine (e.g., XCL1, XCL2), any CX3C chemokine (e.g., CX3CL1)), tumor necrosis factor (e.g., TNF-α, TNF-β)), negative regulators (e.g., PD-1, IL-T) and / or any of the cellular components (e.g., kinases, lipases, nucleases, transcription-related factors (e.g., IRF-1, IRF-7, STAT-5, NFKB, STAT3, STAT1, IRF-10), and / or cell surface markers suppressed or induced by such immunomodulators) involved in the expression of such immunomodulators. The presence, absence or altered expression may be detected within cells of interest or near those cells (e.g., within a cell culture supernatant, nearby cell or tissue *in vitro* or *in vivo,* and / or in blood or plasma). Administration of the immunogen may induce (e.g., stimulate a *de novo* or previously undetected response), or enhance or suppress an existing response against the immunogen by, for example, causing an increased antibody response (e.g., amount of antibody, increased affinity / avidity) or an increased cellular response (e.g., increased number of activated T cells, increased affinity / avidity of T cell receptors, cytoxicity including but not limited to antibody-dependnet cellular cytotoxicity (ADCC), proliferation). In the case of HIV infections, no clear correlates of immunity have been associated with immunity (especially protective immunity), but any of the measures described herein may be helpful in determining the usefulness of the composition and methods described herein. Some immune responses may, in the case of a viral immunogen, lead to decreased viral load in, or lead to elimination of the virus from, a host. In certain embodiments, the immune response may be protective, meaning that the immune response may be capable of preventing initiation or continued infection of or growth within a host and / or by eliminating an agent (e.g., a causative agent, such as HIV) from the host. In some instances, elimination of an agent from the host may mean that the vaccine is therapeutic. In some embodiments, a composition comprising an immunogen may be administered to a population of hosts (e.g., human beings) and determined to provide protective immunity to only a portion of that population. The composition may therefore be considered to protect a portion of that population (e.g., about 1/10, 1/4, 1/3, 1/2, or 3/4 of the population). The proportion of the population that is protected may be calculated and thereby provide the efficacy of the composition in that population (e.g., about 10%, 25%, 33%, 50%, or 75% efficacy).

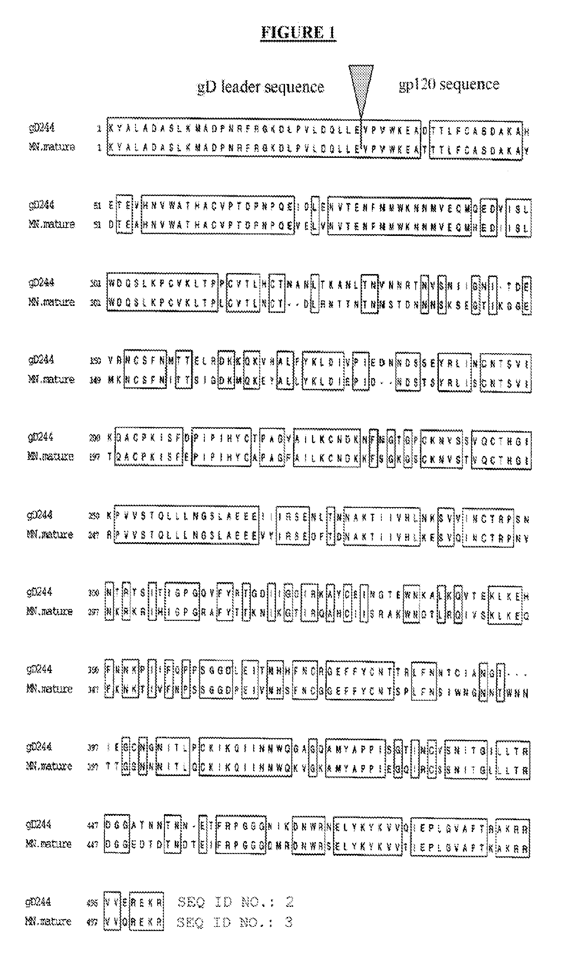

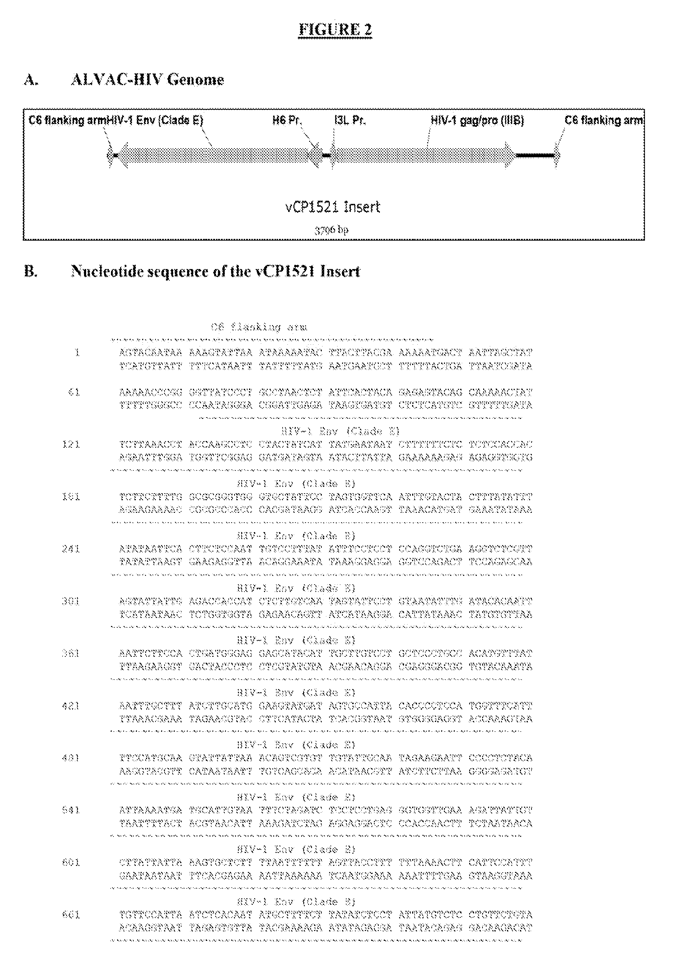

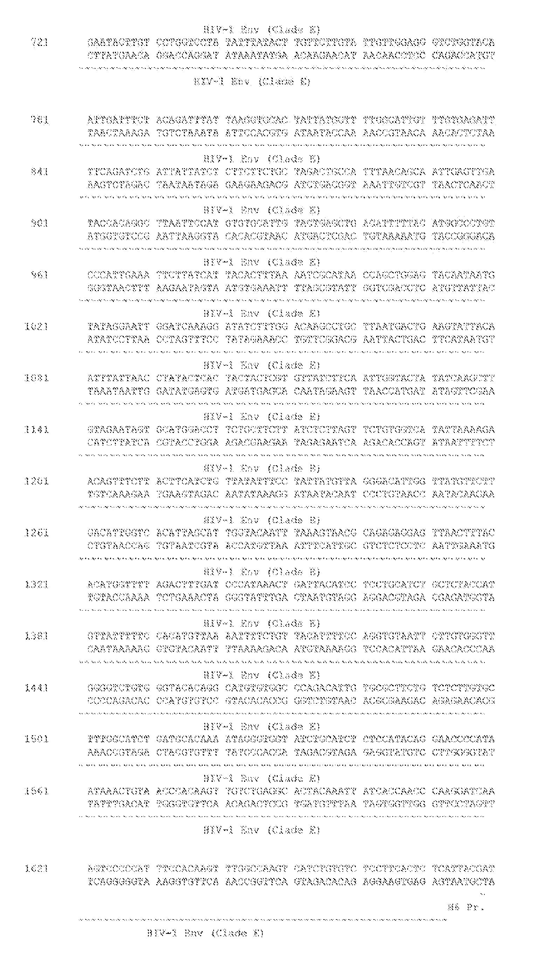

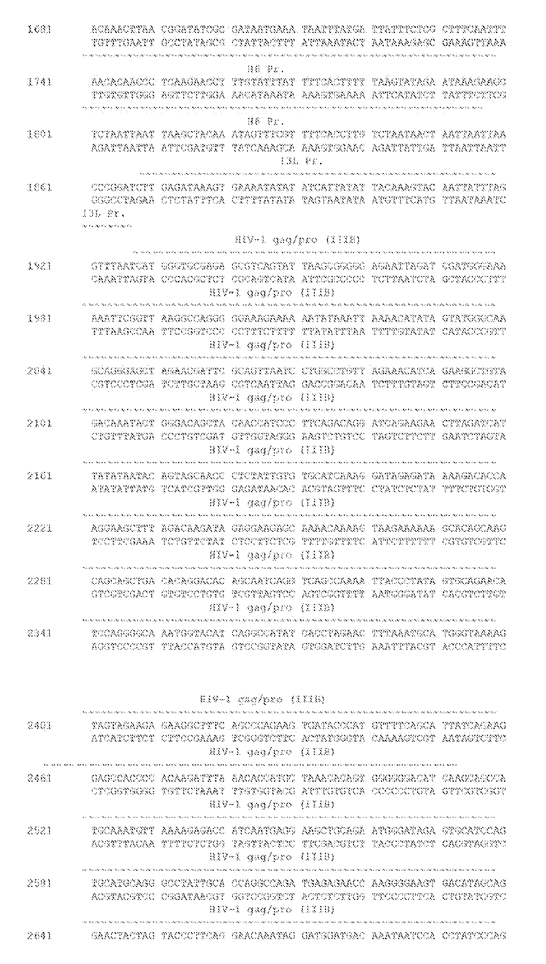

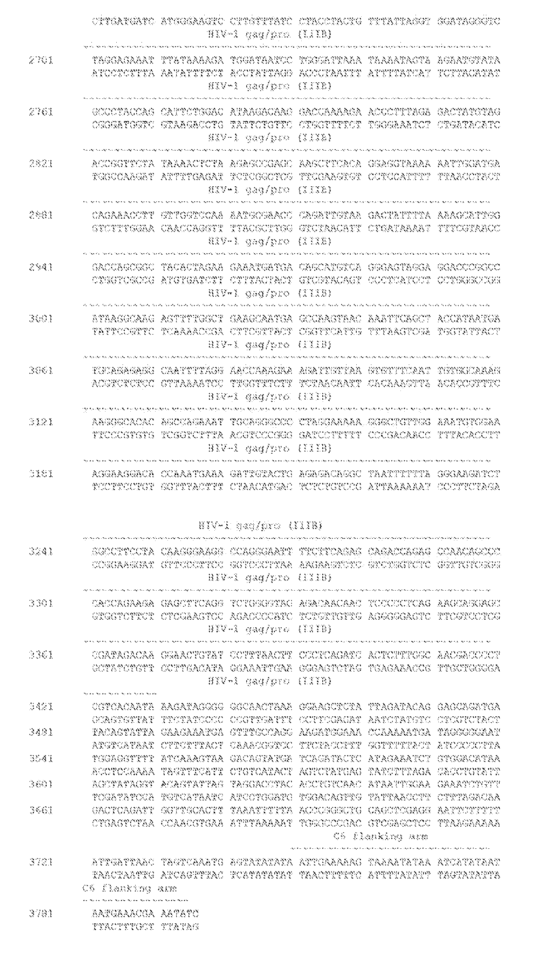

With respect to HIV, immunogens may be selected from any HIV isolate (e.g., any primary or cultured HIV-1, HIV-2, and / or HIV-3 isolate, strain, or clade). As is well-known in the art, HIV isolates are now classified into discrete genetic subtypes. HIV-1 is known to comprise at least ten subtypes (A1, A2, A3, A4, B, C, D, E, F1, F2, G, H, J and K) (Taylor et al, NEJM, 359(18):1965-1966 (2008)). HIV-2 is known to include at least five subtypes (A, B, C, D, and E). Subtype B has been associated with the HIV epidemic in homosexual men and intravenous drug users worldwide. Most HIV-1 immunogens, laboratory adapted isolates, reagents and mapped epitopes belong to subtypes B. In sub-Saharan Africa, India, and China, areas where the incidence of new HIV infections is high, HIV-1 subtype B accounts for only a small minority of infections, and subtype HIV-1 C appears to be the most common infecting subtype. Thus, in certain embodiments, it may be preferable to select immunogens from particular subtypes (e.g., HIV-1 subtypes B and / or C). It may be desirable to include immunogen from multiple HIV subtypes (e.g., HIV-1 subtypes B and C, HIV-2 subtypes A and B, or a combination of HIV-1, HIV-2, and/or HIV-3 subtypes) in a single immunological composition. Suitable HIV imnumogens include HIV envelope (env; e.g., NCBI Ref. Seq. NP_057856, or as shown in any of **Figs. 1****,****2** and / or SEQ ID NOS. 1-6), gag (e.g., p6, p7, p17, p24, GenBank AAD39400.1), the protease encoded by *pol* (e.g., UniProt P03366), nef (e.g., GenBank CAA41585.1; Shugars, et al. J. Virol, Aug. 1993, pp. 4639-4650 (1993)), as well as variants, derivatives, and fusion proteins thereof, as described by, for example, Gomez et al. Vaccine, Vol. 25, pp. 1969-1992 (2007). Immunogens (e.g., env and pol) may be combined as desired. Immunogens from different HIV isolates (e.g., HIV-1 A1 env and HIV-1 A2 env) may also be combined (e.g., AIDSVAX B/B™ and AIDSVAX™ B/E). In some embodiments, at least one of the compositions comprises an amino acid sequence corresponding to that of HIV and at least one amino acid sequence corresponding to a herpes simplex virus (HSV). In some embodiments, the HSV antigen may be the glycoprotein D (gD) leader sequence shown in **Fig. 1****.** In certain embodiments, the HSV amino acid sequence comprises KYALADASLKMADPNRFRGKDLPVLDQL (SEQ ID NO. 7), or a fragment or derivative thereof. In some embodiments, at least one of the compositions comprises SEQ ID NO. 7. In some embodiments, a suitable immunogen may be the polypeptide gd244 as shown in **Fig. 1****,** or a fragment thereof. In others, a suitable immunogen may be the polypeptide "MN.mature" shown in **Fig. 1****,** or a fragment thereof. Suitable strains and combinations may be selected by the skilled artisan as desired.

In some embodiments, a method for protectively immunizing a human being against human immunodeficiency virus (HIV) by administering to the human being at least one dose of a first composition comprising a viral vector encoding an HIV polypeptide or fragment or derivative thereof and subsequently administering to the human being at least one dose of a second composition comprising the HIV polypeptide or fragment or derivative thereof, wherein a protective immune response directed against HIV results, is provided. In some embodiments, a method for protectively immunizing a human being against human immunodeficiency virus (HIV) by administering to the human being a vaccine consisting essentially of a first composition and a second composition, the first composition consisting essentially of a live, attenuated viral vector encoding at least one HIV gp120 or fragment or derivative thereof and, optionally, at least one additional HIV polypeptide or fragment or derivative thereof, the second composition consisting essentially of at least one HIV gp120 polypeptide or fragment or derivative thereof and, optionally, at least one additional HIV polypeptide or fragment or derivative thereof; the method comprising the steps of administering the first composition to the human being and subsequently administering to the human being at least one composition or combination of compositions selected from the group consisting of: the second composition alone; the first and second compositions, optionally separately or together as a single dose; at least one additional dose of the first composition followed by at least one dose of the second composition; the second composition followed by at least one additional dose of the first composition, optionally followed by at least one additional dose of the first and / or second composition; wherein a protective immune response against HIV is induced in the human being, is provided. In some embodiments, the compositions are administered together (e.g., at essentially the same time (e.g., simultaneously) to the same or different sites of a host) or separately (e.g., either in time or site of administration in the host).

In certain embodiments, at least one of the compositions comprises an amino acid sequence corresponding to that of a herpes simplex virus (HSV) (e.g., glycoprotein D (gD) leader sequence shown in **Fig. 1**)**.** In certain embodiments, the HSV amino acid sequence may include, for example, KYALADASLKMADPNRFRGKDLPVLDQL (SEQ ID NO. 7), or a fragment thereof. In some embodiments, the HSV amino acid sequence may be that shown in the polypeptide "MN.matare" shown in **Fig. 1** (SEQ ID NO.:3), or a fragment thereof. In some embodiments, the HSV amino acid sequence may be that shown in the polypeptide gd244 as shown in **Fig.** 1 (SEQ ID NO.:4), or a fragment thereof. In some embodiments, the gp 120 amino acid sequence may be SEQ ID NO.: 5. In some embodiments, at least one of the compositions may comprise a polypeptide of any one or more of SEQ ID NOS. 3, 4, 5, or 7. By "comprise a polypeptide" is meant both a polypeptide per se and / or one encoded by a nucleic acid contained within an expression vector. Variations and derivatives of the polypeptides referred to herein may also be suitable, as could be determined by one of skill in the art.

In some embodiments, the first composition is administered repeatedly prior to at least one administration of the second composition, where the time between administrations is of sufficient length to allow for the development of an immune response within the human beings. In some embodiments, the methods described herein comprise administering the vaccine is administered to a population of human beings such that at least about one-third of that population is protected from infection by HIV. In some embodiments, the first composition is administered repeatedly prior to at least one administration of the second composition, with the time between administrations is of sufficient length to allow for the development of an immune response within the human being. In certain embodiments, administration of either or both the first and second compositions is via a route selected from the group consisting of mucosal, intradermal, intramuscular, subcutaneous, via skin scarification, intranodal, or intratumoral. The dose of the compositions may vary, but in some embodiments, the amount of viral vector administered in each dose is the equivalent of about 10⁷ CCID₅₀ and the total amount of polypeptide administered in each dose is about 600 µg. In some embodiments, the viral vector may be a poxviral vector such as vaccinia, NYVAC, Modified Virus Ankara (MVA), avipox, canarypox, ALVAC, ALVAC(2), fowlpox, or TROVAC. In some embodiments, the viral vector may be ALVAC-HIV (vCP1521). The viral vector may comprise the nucleic acid sequence of SEQ ID NO. 1 or 5, for example. The second composition may be AIDSVAX^{®} B/B or AIDSVAX^{®} B/E. In some embodiments, the viral vector may be ALVAC-HIV (vCP1521) and the second composition may be AIDSVAX^{®} B/E.

In certain embodiments, the HIV polypeptide or HIV gp120 is derived from an HIV virus selected from the group consisting of HIV-1, HIV-2, and HIV-3, wherein the first and second composition contain the same or different HIV polypeptides and / or gp120. The HIV-1 may be, for example, HIV-1 subtype A1, HIV-1 subtype A2, HIV-1 subtype A3, HIV-1 subtype A4, HIV-1 subtype B, HIV-1 subtype C, HIV-1 subtype D, HIV-1 subtype E, HIV-1 subtype F1, HIV-1 subtype F2, HIV-1 subtype G, HIV-1 subtype H, HIV-1 subtype J and HlV-1 subtype K. The HIV-2 may be, for example, HIV-2 subtype A, HIV-2 subtype B, HIV-2 subtype C, HIV-2 subtype D, and HIV-2 subtype E. The viral vector may encode, for example, at least one polypeptide selected from the group consisting of HIV gp120 MN 12-485, HIV gp120 A244 12-484, and HIV gp120 GNE8 12-477.

Where multiple HIV immunogens are used, the at least one additional HIV immunogen may be, for example, gag, pol, nef, a variant thereof, and a derivative thereof. Thus, is some embodiments, the first or second composition additionally contain at least one additional HIV immunogen selected from the group consisting of gag the protease component encoded by pol, nef, a variant thereof, and a derivative thereof

In preferred embodiments of the present invention, vectors are used to transfer a nucleic acid sequence encoding a polypeptide to a cell. A vector is any molecule used to transfer a nucleic acid sequence to a host cell. In certain cases, an expression vector is utilized. An expression vector is a nucleic acid molecule that is suitable for transformation of a host cell and contains nucleic acid sequences that direct and / or control the expression of the transferred nucleic acid sequences. Expression includes, but is not limited to, processes such as transcription, translation, and splicing, if introns are present. Expression vectors typically comprise one or more flanking sequence operably linked to a heterologous nucleic acid sequence encoding a polypeptide. As used herein, the term operably linked refers to a linkage between polynucleotide elements in a functional relationship such as one in which a promoter or enhancer affects transcription of a coding sequence. Flanking sequences may be homologous (i.e., from the same species and/or strain as the host cell), heterologous (i.e., from a species other than the host cell species or strain), hybrid (i.e., a combination of flanking sequences from more than one source), or synthetic, for example.

In certain embodiments, it is preferred that the flanking sequence is a trascriptional regulatory region that drives high-level gene expression in the target cell. The transcriptional regulatory region may comprise, for example, a promoter, enhancer, silencer, repressor element, or combinations thereof The transcriptional regulatory region may be either constitutive, tissue-specific, cell-type specific (i.e., the region is drives higher levels of transcription in a one type of tissue or cell as compared to another), or regulatable (i.e., responsive to interaction with a compound such as tetracycline). The source of a transcriptional regulatory region may be any prokaryotic or eukaryotic organism, any vertebrate or invertebrate organism, or any plant, provided that the flanking sequence functions in a cell by causing transcription of a nucleic acid within that cell. A wide variety of transcriptional regulatory regions may be utilized in practicing the present invention.

In some embodiments, derivatives of polypeptides, peptides, or polynucleotides incorporated into or expressed by the vectors described herein including, for example, fragments and / or variants thereof may be utilized. Derivatives may result from, for example, substitution, deletion, or addition of amino acids or nucleotides from or to the reference sequence (e.g., the parental sequence). A derivative of a polypeptide or protein, for example, typically refers to an amino acid sequence that is altered with respect to the referenced polypeptide or peptide. A derivative of a polypeptide typically retains at least one activity of the polypeptide. A derivative will typically share at least approximately 60%, 70%, 80%, 90%, 95%, or 99% identity to the reference sequence. With respect to polypeptides and peptides, the derivative may have "conservative" changes, wherein a substituted amino acid has similar structural or chemical properties. A derivative may also have "nonconservative" changes. Exemplary, suitable conservative amino acid substitutions may include, for example, those shown in **Table 1:**

**Table 1**

| Original Residues | Exemplary Substitutions | Preferred Substitutions |
|---|---|---|
| Ala | Val, Leu, Ile | Val |
| Arg | Lys, Gln, Asn | Lys |
| Asn | Gln | Gln |
| Asp | Gln | Glu |
| Cys | Ser, Ala | Ser |
| Gln | Asn | Asn |
| Glu | Asp | Asp |
| Gly | Pro, Ala | Ala |
| His | Asn, Gln, Lys, Arg | Arg |
| Ile | Leu, Val, Met, Ala, Phe, Norleucine | Leu |
| Leu | Norleucine, Ile, Val, Met, Ala, Phe | Ile |
| Lys | Arg, 1,4 Diamino-butyric Acid, Gln, Asn | Arg |
| Met | Leu, Phe, Ile | Leu |
| Phe | Leu, Val, Ile, Ala, Tyr | Leu |
| Pro | Ala | Gly |
| Ser | Thr, Ala, Cys | Thr |
| Thr | Ser | Ser |
| Trp | Tyr, Phe | Tyr |
| Tyr | Trp, Phe, Thr, Ser | Phe |
| Val | Ile, Met, Leu, Phe, Ala, Norleucine | Leu |

Other amino acid substitutions may be considered non-conservative. Derivatives may also include amino acid or nucleotide deletions and / or additions / insertions, or some combination of these. Guidance in determining which amino acid residues or nucleotides may be substituted, inserted, or deleted without abolishing die desired activity of the derivative may be identified using any of the methods available to one of skill in the art.

Derivatives may also refer to a chemically modified polynucleotide or polypeptide. Chemical modifications of a polynucleotide may include, for example, replacement of hydrogen by an alkyl, acyl, hydroxyl, or amino group. A derivative polynucleotide may encode a polypeptide which retains at least one biological or immunological function of the natural molecule. A derivative polypeptide may be one modified by glycosylation, pegylation, biotinylation, or any similar process that retains at least one biological or immunological function of the polypeptide from which it was derived.

The phrases "percent identity" and "% identity," as applied to polypeptide sequences, refer to the percentage of residue matches between at least two polypeptide sequences aligned using a standardized algorithm. Methods of polypeptide sequence alignment are well-known. Some alignment methods take into account conservative amino acid substitutions. Such conservative substitutions, explained in more detail above, generally preserve the charge and hydrophobicity at the site of substitution, thus preserving the structure (and therefore function) of the polypeptide. Percent identity may be measured over the length of an entice defined polypeptide sequence, for example, as defined by a particular SEQ ID number, or may be measured over a shorter length, for example, over the length of a fragment taken from a larger, defmed polypeptide sequence, for instance, a fragment of at least 10, at least 15, at least 20, at least 30, at least 40, at least 50, at least 70 or at least 150 contiguous residues. Such lengths are exemplary only, and it is understood that any fragment length supported by the sequences shown herein, in the tables, figures or Sequence Listing, may be used to describe a length over which percentage identity may be measured. Percent identity can be measured both globally or locally. Examples of alignment algorithms known in the art for global alignments are ones which attempt to align every residue in every sequence, such as the Needleman-Wunsch algorithm. Local alignment algorithms are useful for dissimilar sequences that contain regions of similar sequence motifs within their larger sequence, such as the Smith-Waterman algorithm.

As mentioned above, this disclosure relates to compositions comprising recombinant vectors, the vectors per se, and methods of using the same. A "vector" is any moiety (e.g., a virus or plasmid) used to carry, introduce, or transfer a polynucleotide or interest to another moiety (e.g., a host cell). In certain cases, an expression vector is utilized. An expression vector is a nucleic acid molecule containing a polynucleotide of interest encoding a polypeptide, peptide, or polynucleotide and also containing other polynucleotides that direct and / or control the expression of the polynucleotide of interest. Expression includes, but is not limited to, processes such as transcription, translation, and / or splicing (e.g., where introns are present).

Viral vectors that may be used includes, for example, retrovirus, adenovirus, adeno-associated virus (AAV), alphavirus, herpes virus, and poxvirus vectors, among others. Many such viral vectors are available in the art. The vectors described herein may be constructed using standard recombinant techniques widely available to one skilled in the art. Such techniques may be found in common molecular biology references such as Molecular Cloning A Laboratory Manual (Sambrook, et al., 1989, Cold Spring Harbor Laboratory Press), Gene Expression Technology (Methods in Enzymology, Vol. 185, edited by D. Goeddel, 1991. Academic Press, San Diego, CA), and PCR Protocols: A Guide to Methods and Applications (Innis, et al. 1990. Academic Press, San Diego, CA).

Suitable retroviral vectors may include derivatives of lentivirus as well as derivatives of murine or avian retroviruses. Examplary, suitable retroviral vectors may include, for example, Moloney murine leukemia virus (MoMnLV), Harvey murine sarcoma virus (HaMuSV), murine mammary tumor virus (MuMTV), SIV, BIV, HIV and Rous Sarcoma Virus (RSV). A number of retroviral vectors can incorporate multiple exogenous polynucleotides. As recombinant retroviruses are defective, they require assistance in order to produce infectious vector particles. This assistance can be provided by, for example, helper cell lines encoding retrovirus structural genes. Suitable helper cell lines include Ψ2, PA317 and PA12, among others. The vector virions produced using such cell lines may then be used to infect a tissue cell line, such as NIH 3T3 cells, to produce large quantities of chimeric retroviral virions. Retroviral vectors may be administered by traditional methods (i.e., injection) or by implantation of a "producer cell line" in proximity to the target cell population (Culver, K., et al., 1994, Hum. Gene Ther., 5 (3): 343-79; Culver, K., et al., Cold Spring Harb. Symp. Quant. Biol., 59: 685-90); Oldfield, E., 1993, Hum. Gene Ther., 4 (1): 39-69). The producer cell line is engineered to produce a viral vector and releases viral particles in the vicinity of the target cell. A portion of the released viral particles contact the target cells and infect those cells, thus delivering a nucleic acid encoding an immunogen to the target cell. Following infection of the target cell, expression of the polynucleotide of interest from the vector occurs.

Adenoviral vectors have proven especially useful for gene transfer into eukaryotic cells (Rosenfeld, M., et al., 1991, Science, 252 (5004): 431-4; Crystal, R., et al., 1994, Nat. Genet., 8 (1): 42-51), the study eukaryotic gene expression (Levrero, M., et al., 1991, Gene, 101 (2): 195-202), vaccine development (Graham, F. and Prevec, L, 1992, Biotechnology, 20: 363-90), and in animal models (Stratford-Perricaudet, L., et al., 1992, Bone Marrow Transplant., 9 (Suppl. 1). 151-2 ; Rich, et al., 1993, Hum. Gene Ther., 4 (4): 461-76). Experimental routes for administrating recombinant Ad to different tissues *in vivo* have included intratracheal instillation (Rosenfeld, M., et al., 1992, Cell, 68 (1): 143-55) injection into muscle (Quantin, B., et al., 1992, Proc. Natl. Acad. Sci. U.S.A., 89 (7): 2581-4), peripheral intravenous injection (Herz, J., and Gerard, R., 1993, Proc. Natl. Acad. Sci U.S.A., 90 (7): 2812-6) and / or stereotactic inoculation to brain (Le Gal La Salle, G., et al., 1993, Science, 259 (5097): 988-90), among others.

Adeno-associated virus (AAV) demonstrates high-level infectivity, broad host range and specificity in integrating into the host cell genome (Hermonat, P., et al., 1984, Proc. Natl. Acad. Sci. U.S.A., 81 (20): 6466-70). And Herpes Simplex Virus type-1 (HSV-1) is yet another attractive vector system, especially for use in the nervous system because of its neurotropic property (Geller, A., et al., 1991, Trends Neurosci., 14 (10): 428-32; Glorioso, et al., 1995, Mol. Biotechnol., 4 (1): 87-99; Glorioso, et al., 1995, Annu. Rev. Microbiol., 49:675-710).

Alphavirus may also be used to express the immunogen in a host. Suitable members of the Alphavirus genus include, among others, Sindbis virus, Semliki Forest virus (SFV), the Ross River virus and Venezuelan, Western and Eastern equine encephalitis viruses, among others. Expression systems utilizing alphavirus vectors are described in, for example, U.S. Pat. Nos. 5,091,309; 5,217,879; 5,739,026; 5,766,602; 5,843,723; 6,015,694; 6,156,558; 6,190,666; 6,242,259; and, 6,329,201; WO 92/10578; Xiong et al., Science, Vol 243, 1989, 1188-1191; Liliestrom, et al. Bio/Technology, 9: 13.56-1361, 1991. Thus, the use of alphavirus as an expression system is well known by those of skill in the art.

Poxvirus is another useful expression vector (Smith, et al. 1993, Gene, 25 (1): 21-8; Moss, et al, 1992, Biotechnology, 20:345-62; Moss, et al, 1992, Curr. Top. Microbiol. Immunol, 158: 25-38; Moss, et al. 1991. Science, 252: 1662-1667). The most often utilized poxviral vectors include vaccinia and derivatives therefrom such as NYVAC and MVA, and members of the avipox genera such as fowlpox, canarypox, ALVAC, and ALVAC(2), among others.

An exemplary suitable vector is NYVAC (vP866) which was derived from the Copenhagen vaccine strain of vaccinia virus by deleting six nonessential regions of the genome encoding known or potential virulence factors (see, for example, U.S. Pat. Nos. 5,364,773 and 5,494,807). The deletion loci were also engineered as recipient loci for the insertion of foreign genes. The deleted regions are: thymidine kinase gene (TK; J2R); hemorrhagic region (u; B13R+B14R), A type inclusion body region (ATI; A26L); hemagglutinin gene (HA; A56R); host range gene region (C7L-K1L); and, large subunit, ribonucleotide reductase (I4L). NYVAC is a genetically engineered vaccinia virus strain that was generated by the specific deletion of eighteen open reading frames encoding gene products associated with virulence and host range. NYVAC has been show to be useful for expressing TAs (see, for example, U,S. Pat No. 6,265,189). NYVAC (vP866), vP994, vCP205, vCP1433, platZH6H4Lreverse, pMPC6H6K3E3 and pC3H6FHVB were also deposited with the ATCC under the terms of the Budapest Treaty, accession numbers VR-2559, VR-2558, VR-2557, VR-2556, ATCC-97913, ATCC-97912, and ATCC-97914, respectively.

Another suitable virus is the Modified Vaccinia Ankara (MVA) virus which was generated by 516 serial passages on chicken embryo fibroblasts of the Ankara strain of vaccinia virus (CVA) (for review, see Mayr, A., et al. Infection 3, 6-14 (1975)). It was shown in a variety of animal models that the resulting MVA was significantly avirulent (Mayr, A. & Danner, K. (1978) Dev. Biol. Stand. 41: 225.34) and has been tested in clinical trials as a smallpox vaccine (Mayr et al., Zbl. Bakt. Hyg. I, Abt. Org. B 167, 375-390 (1987), Stickl et al., Dtsch. med. Wschr. 99, 2386-2392 (1974)). MVA has also been engineered for use as a viral vector for both recombinant gene expression studies and as a recombinant vaccine (Sutter, G. et al. (1994), Vaccine 12: 1032-40; Blanchard et al., 1998, J Gen Virol 79, 1159-1167; Carroll & Moss, 1997, Virology 238, 198-211; Altenberger, U.S. Pat. No. 5,185,146; Ambrosmi et al., 1999, J Neurosci Res 55(5), 569). Modified virus Ankara (MVA) has been previously described in, for example, U.S. Pat. Nos. 5,185,146 and 6,440,422; Sutter, et al. (B. Dev. Biol. Stand. Basel, Karger 84:195-200 (1995)); Antoine, et al. (Virology 244: 365-396, 1998); Sutter et al. (Proc. Natl. Acad. Sci. USA 89: 10847-10851, 1992); Meyer et al. (J. Gen. Virol. 72: 1031-1038, 1991); Mahnel, ett al. (Berlin Munch. Tierarztl. Wochenschr. 107: 253-256, 1994); Mayr et al. (Zbl. Bakt. Hyg. I, Abt. Org. B 167: 375-390 (1987); and, Stickl et al. (Dtsch. med. Wschr. 99: 2386-2392 (1974)). An exemplary MVA is available from the ATCC under accession numbers VR-1508 and VR-1566.

ALVAC-based recombinant viruses (i.e., ALVAC-1 and ALVAC-2) are also suitable for use in practicing the present invention (see, for example, U.S. Pat. No. 5,756,103). ALVAC(2) is identical to ALVAC(1) except that ALVAC(2) genome comprises the vaccinia E3L and K3L genes under the control of vaccinia promoters (U.S. Pat. No. 6,130,066; Beattie et al., 1995a, 1995b, 1991; Chang et al., 1992; Davies et al., 1993). Both ALVAC(1) and ALVAC(2) have been demonstrated to be useful in expressing foreign DNA sequences, such as TAs (Tartaglia et al., 1993 a,b; U.S. Pat. No. 5,833,975). ALVAC was deposited under the terms of the Budapest Treaty with the American Type Culture Collection (ATCC), 10801 University Boulevard, Manassas, Va. 20110-2209, USA, ATCC accession number VR-2547. Vaccinia virus host range genes (e.g., C18L, G17L, C7L, KIL, E3L, B4R, B23R, and B24R) have also been shown to be expressible in canarypox (e.g., U.S. Pat. No. 7,473,536).

Another useful poxvirus vector is TROVAC. TROVAC refers to an attenuated fowlpox that was a plaque-cloned isolate derived from the FP-1 vaccine strain of fowlpoxvirus which is licensed for vaccination of 1 day old chicks. TROVAC was likewise deposited under the terms of the Budapest Treaty with the ATCC, accession number 2553.

"Non-viral" plasmid vectors may also be suitable for use. Plasmid DNA molecules comprising expression cassettes for expressing an immunogen may be used for "naked DNA" immunization. Preferred plasmid vectors are compatible with bacterial, insect, and / or mammalian host cells. Such vectors include, for example, PCR-II, pCR3, and pcDNA3.1 (Invitrogen, San Diego, CA), pBSII (Stratagene, La Jolla, CA), pET15 (Novagen, Madison, WI), pGEX (Pharmacia Biotech, Piscataway, NJ), pEGFP-N2 (Clontech, Palo Alto, CA), pETL (BlueBacII, Invitrogen), pDSR-alpha (PCT pub. No. WO 90/14363) and pFastBacDual (Gibco-BRL, Grand Island, NY) as well as Bluescript^{®} plasmid derivatives (a high copy number COLE1-based phagemid, Stratagene Cloning Systems, La Jolla, CA), PCR cloning plasmids designed for cloning Taq-amplified PCR products (*e*.*g*., TOPO™ TEA cloning^{®} kit, PCR2.1^{®} plasmid derivatives Invitrogen, Carlsbad, CA).

Bacterial vectors may also be suitable for use. These vectors include, for example, Shigella, Salmonella (e.g., Darji, et al. Cell, 91: 765-775 (1997); Woo, et al. Vaccine, 19: 2945-2954 (2001)), Vibrio cholerae, Lactobacillus, Bacille calmette guérin (BCG), and Streptococcus (e.g., WO 88/6626, WO 90/0594, WO 91/13157, WO 92/1796, and WO 92/21376). Many other non-viral plasmid expression vectors and systems are known in the art and could be used with the current invention.

Nucleic acid delivery or transformation techniques that may be used include DNA-ligand complexes, adenovirus-ligand-DNA complexes, direct injection of DNA, CaPO₄ precipitation, gene gun techniques, electroporation, and colloidal dispersion systems, among others. Colloidal dispersion systems include macromolecule complexes, nanocapsules, microspheres, beads, and lipid-based systems including oil-in-water emulsions, micelles, mixed micelles, and liposomes. The preferred colloidal system of this invention is a liposome, which are artificial membrane vesicles useful as delivery vehicles *in vitro* and *in vivo.* RNA, DNA and intact virions can be encapsulated within the aqueous interior and be delivered to cells in a biologically active form (Fraley, R., et al. Trends Biochem. Sci., 6: 77 (1981)). The composition of the liposome is usually a combination of phospholipids, particularly high-phase-transition-temperature phospholipids, usually in combination with steroids, especially cholesterol. Other phospholipids or other lipids may also be used. The physical characteristics of liposomes depend on pH, ionic strength, and the presence of divalent cations. Examples of lipids useful in liposome production include phosphatidyl compounds, such as phosphatidylglycerol, phosphatidylcholine, phosphatidylserine, phosphatidylethanolamine, sphingolipids, cerebrosides, and gangliosides. Particularly useful are diacylphosphatidylglycerols, where the lipid moiety contains from 14-18 carbon atoms, particularly from 16-18 carbon atoms, and is saturated. Illustrative phospholipids include egg phosphatidylcholine, dipalmitoylphosphatidylcholine and distearoylphosphatidylcholine.

Strategies for improving the efficiency of nucleic acid-based immunization may also be used including, for example, the use of self-replicating viral replicons (Caley, et al. Vaccine, 17: 3124-2135 (1999); Dubensky, et al. Mol. Med. 6: 723-732 (2000); Leitner, et al. Cancer Res. 60:51-55 (2000)), codon optimization (Liu, et al. Mol. Ther., 1: 497-500 (2000); Dubensky, supra; Huang, et al. J. Virol. 75: 4947-495 (2001)), in vivo electroporation (Widera, et al. J. Immunol. 164: 4635-3640 (2000)), incorporation of CpG stimulatory motifs (Gurunathan, et al. Ann. Rev. Immunol. 18: 927-974 (2000); Leitner, supra), sequences for targeting of the endocytic or ubiquitin-processing pathways (Thomson, et al. J. Virol. 72: 2246-2252 (1998); Velders, et al. J. Immunol. 166: 5366-5373 (2001)), and / or prime-boost regimens (Gurunathan, supra; Sullivan, et al. Nature, 408:605-609 (2000); Hanke, et al. Vaccine, 16: 439-445 (1998); Amara, et al. Science, 292: 69-74 (2001)). Other methods are known in the art, some of which are described below.

In other embodiments, it may be advantageous to combine or include within the compositions or recombinant vectors additional polypeptides, peptides or polynucleotides encoding one or more polypeptides or peptides that function as "co-stimulatory" component(s). Such co-stimulatory components may include, for example, cell surface proteins, cytokines or chemokines in a composition of the present invention. The co-stimulatory component may be included in the composition as a polypeptide or peptide, or as a polynucleotide encoding the polypeptide or peptide, for example. Suitable co-stimulatory molecules include, for instance, polypeptides that bind members of the CD28 family (i.e., CD28, ICOS; Hutloff, et al. Nature 1999, 397: 263-265; Peach, et al. J Exp Med 1994, 180: 2049-2058) such as the CD28 binding polypepides B7.1 (CD80; Schwartz, 1992; Chen et al, 1992; Ellis, et al. J. Immunol, 156(8): 2700-9) and B7.2 (CD86; Ellis, et al. J. Immunol., 156(8): 2700-9); polypeptides which bind members of the integrin family (i.e., LFA-1 (CD11a / CD18); Sedwick, et al. J Immunol 1999, 162: 1377-1375; Wülfing, et al. Science 1998, 282: 2266-2269; Lub, et al. Immunol Today 1995, 16: 479-483) including members of the ICAM family (i.e., ICAM-1, -2 or -3); polypeptides which bind CD2 family members (i.e., CD2, signalling lymphocyte activation molecule (CDw150 or "SLAM"; Aversa, et al. J Immunol 1997, 158: 4036-4044) such as CD58 (LFA-3; CD2 ligand; Davis, et al. Immunol Today 1996, 17: 177-187) or SLAM ligands (Sayos, et al. Nature 1998, 395: 462-469); polypeptides which bind heat stable antigen (HSA or CD24; Zhou, et al. Eur J Immunol 1997, 27: 2524-2528); polypeptides which bind to members of the TNF receptor (TNFR) family (i.e., 4-1BB (CD137; Vinay, et al. Semin Immunol 1998, 10: 481-489)), OX40 (CD134; Weinberg, et al. Semin Immunol 1998, 10: 471-480; Higgins, et al. J Immunol 1999, 162: 486-493), and CD27 (Lens, et al. Semin Immunol 1998, 10: 491-499)) such as 4.1BBL (4-1BB ligand; Vinay, et al. Semin Immunol 1998, 10:481-48; DeBenedette, et al. J Immumol 1997, 158: 551-559), TNFR associated factor-1 (TRAF-1; 4-1BB ligand; Saoulli, et al. J Exp Med 1998, 187: 1849-1862, Arch, et al. Mol Cell Biol 1998, 18; 558-565), TRAF-2 (4-1BB and OX40 ligand; Saoulli, et al. J Exp Med 1998, 187: 1849-1862; Oshima, et al. Int Immunol 1998, 10: 517-526, Kawamata, et al. J Biol Chem 1998, 273: 5808-5814), TRAF-3 (4-1BB and OX40 ligand; Arch, et al. Mol Cell Biol 1998, 18: 558-565; Jang, et al. Biochem Biophys Res Commun 1998, 242: 613-620; Kawamata S, et al. J Biol Chem 1998, 273: 5808-5814), OX40L (OX40 ligand; Gramaglía, et al. J Immunol 1998, 161: 6510-6317), TRAF-5 (OX40 ligand; Arch et al. Mol Cell Biol 1998, 18: 558-565; Kawamata, et al. J Biol Chem 1998, 273: 5808-5814), and CD70 (CD27 ligand; Couderc, et al. Cancer Gene Ther., 5(3): 163-75). CD154 (CD40 ligand or "CD40L"; Gurunathan, et al. J. Immunol., 1998, 161: 4563-4571; Sine, et al. Hum. Gene Ther., 2001, 12: 1091-1102) Other co-stimulatory molecules may also be suitable for practicing the present invention.

One or more cytokines may also be suitable co-stimulatory components or "adjuvants", either as polypeptides or being encoded by nucleic acids contained within the compositions of the present invention (Parmiani, et al. Immumol Lett 2000 Sep 15; 74(1): 41-4; Berzofsky, et al. Nature Immunol. 1: 209-219). Suitable cytokines include, for example, interleukin-2 (IL-2) (Rosenberg, et al. Nature Med 4: 321-327 (1998)), IL-4, IL-7, IL-12 (reviewed by Pardoll, 1992; Harries, et al. J. Gene Med. 2000 Jul-Aug;2(4):243-9; Rao, et al. J. Immunol. 156: 3357-3365 (1996)), IL-15 (Xin, et al. Vaccine, 17:858-866, 1999), IL-16 (Cruikshank, et al. J. Leuk Biol. 67(6): 757-66, 2000), IL-18 (J. Cancer Res. Clin, Oncol, 2001. 127(12): 718-726), GM-CSF (CSF (Disis, et al. Blood, 88: 202-210 (1996)), tumor necrosis factor-alpha (TNF-α), or interferon-gamma (INF-γ). Other cytokines may also be suitable for practicing the present invention.

Chemokines may also be utilized. For example, fusion proteins comprising CXCL10 (IP-10) and CCL7 (MCP-3) fused to a tumor self-antigen have been shown to induce anti-tumor immunity (Biragyn, et al. Nature Biotech. 1999, 17: 253-258). The chemokines CCL3 (MIP-1α) and CCL5 (RANTES) (Boyer, et al. Vaccine, 1999, 17 (Supp. 2): S53-S64) may also be of use in practicing the present invention. Other suitable chemokines are known in the art.

It is also known in the art that suppressive or negative regulatory immune mechanisms may be blocked, resulting in enhanced immune responses. For instance, treatment with anti-CTLA-4 (Shrikant, et al. Immunity, 1996, 14: 145-155; Satmuller, et al. J. Exp. Med., 2001, 194: 823-832), anti-CD25 (Sutmuller, *supra*), anti-CD4 (Matsui, et al. J. Immunol., 1999, 163: 184-193), the fusion protein IL13Ra2-Fc (Terabe, et al. Nature Immunol., 2000, 1: 515-520), and combinations thereof (i.e., anti-CTLA-4 and anti-CD25, Sutmuller, *supra*) have been shown to upregulate anti-tumor immune responses and would be suitable in practicing the present invention.

An immunogen may also be administered in combination with one or more adjuvants to boost the immune response. Adjuvants may also be included to simulate or enhance the immune response against the immunogen. Non-limiting examples of suitable adjuvants include those of the gel-type (i.e., aluminum hydroxide/phosphate ("alum adjuvants"), calcium phosphate), of microbial origin (muramyl dipeptide (MDP)), bacterial exotoxins (cholera toxin (CT), native cholera toxin subunit B (CTB), E. coli labile toxin (LT), pertussis toxin (PT), CpG oligonucleotides, BCG sequences, tetanus toxoid, monophosphoryl lipid A (MPL) of, for example, *E*. *coli, Salmonella minnesota*, *Salmonella typhimurium,* or *Shigella exseri*), particulate adjuvants (biodegradable, polymer microspheres), immunostimulatory complexes (ISCOMs)), oil-emulsion and surfactant-based adjuvants (Freund's incomplete adjuvants (FIA), microfluidized emulsions (MF59, SAF), saponins (QS-21)), synthetic (muramyl peptide derivatives (murabutide, threony-MDP), nonionic block copolymers (L121), polyphosphazene (PCCP), synthetic polynucleotides (poly A:U, poly I:C), thalidomide derivatives (CC-4407/ACTIMID)), RH3-ligand, or polylactide glycolide (PLGA) microspheres, among others. Fragments, homologs, derivatives, and fusions to any of these toxins are also suitable, provided that they retain adjuvant activity. Suitable mutants or variants of adjuvants are described, e.g., in WO 95/17211 (Arg-7- Lys GT mutant), WO 96/6627 (Arg-192-Gly LT mutant), and WO 95/34323 (Arg-9-Lys and Glu-129-Gly PT mutant). Additional LT mutants that can be used in the methods and compositions of the invention include, e. g., Sec-63-Lys, Ala-69-Gly, Glu-110-Asp, and Glu-112-Asp mutants. Other suitable adjuvants are also well-known in the art.

As an example, metallic salt adjuvants such alum adjuvants are well-known in the art as providing a safe excipient with adjuvant activity. The mechanism of action of these adjuvants are thought to include the formation of an antigen depot such that antigen may stay at the site of injection for up to 3 weeks after administration, and also the formation of antigen/metallic salt complexes which are more easily taken up by antigen presenting cells. In addition to aluminium, other metallic salts have been used to adsorb antigens, including salts of zinc, calcium, cerium, chromium, iron, and berylium. The hydroxide and phosphate salts of aluminium are the most common. Formulations or compositions containing aluminium salts, antigen, and an additional immunostimulant are known in the art. An example of an immunostimulant is 3-de-O-acylated monophosphoryl lipid A (3D-MPL).

Any of these components may be used alone or in combination with other agents. For instance, it has been shown that a combination of CD80, ICAM-1 and LFA-3 ("TRICOM") may potentiate anti-cancer immune responses (Hodge, et al. Cancer Res. 59: 5800-5807 (1999). Other selective combinations include, for example, IL-12 + GM-CSF (Ahlers, et al. J. Immunol., 158:3947-3958(1997); Iwasaki, et al, J. Immunol 158: 4591-4601 (1997)), IL-12 + GM-CSF + TNF-*α* (Ahlers, et al. Int. Immunol. 13: 897-908 (2001)), CD80 + IL-12 (Fruend, et al. Int. J. Cancer, 85: 508-517 (2000); Rao, et al. *supra*), and CD86 + GM-CSF + IL-12 (Iwasaki, supra). One of skill in the art would be aware of additional combinations useful in carrying out the present invention. In addition, the skilled artisan would be aware of additional reagents or methods that may be used to modulate such mechanisms. These reagents and methods, as well as others known by those of skill in the art, may be utilized in practicing the present invention.

Other agents that may be utilized in conjunction with the compositions and methods provided herein include anti-HIV agents including, for example, protease inhibitor, an HIV entry inhibitor, a reverse transcriptase inhibitor, and / or or an anti-retroviral nucleoside analog. Suitable compounds include, for example, Agenerase (amprenavir), Combivir (Retrovir / Epivir), Crixivan (indinavir), Emtriva (emtricitabine), Epivir (3tc / lamivudine), Epzicom, Fortovase / Invirase (saquinavir), Fuzeon (enfuvirtide), Hivid (ddc / zalcitabine), Kaletra (lopinavir), Lexiva (Fosamprenavir), Norvir (ritonavir), Rescriptor (delavirdine), Retrovir / AZT (zidovadine), Reyatax (atazanavir, BMS-232632), Sustiva (efavirenz), Trizivir (abacavir / zidovudine / lamivudine), Truvada (Emtricitabine / Tenofovir DF), Videx (ddl / didanosine), Videx EC (ddl, didanosine), Viracept (nevirapine), Viread (tenofovir disoproxil fumarate), Zerit (d4T / stavudine), and Ziagen (abacavir). Other suitable agents are known to those of skill in the art. Such agents may either be used prior to, during, or after administration of the compositions and / or use of the methods described herein.

Administration of a composition of the present invention to a host may be accomplished using any of a variety of techniques known to those of skill in the art. The composition(s) may be processed in accordance with conventional methods of pharmacy to produce medicinal agents for administration to patients, including humans and other mammals (i.e., a "pharmaceutical composition"). The pharmaceutical composition is preferably made in the form of a dosage unit containing a given amount of DNA, viral vector particles, polypeptide, peptide, or other drug candidate, for example. A suitable daily dose for a human or other mammal may vary widely depending on the condition of the patient and other factors, but, once again, can be determined using routine methods. The compositions are administered to a patient in a form and amount sufficient to elicit a therapeutic effect. Amounts effective for this use will depend on various factors, including for example, the particular composition of the vaccine regimen administered, the manner of administrations, the stage and severity of the disease, the general state of health of the patient, and the judgment of the prescribing physician. The dosage regimen for immunizing a host or otherwise treating a disorder or a disease with a composition of this invention is based on a variety of factors, including the type of disease, the age, weight, sex, medical condition of the patient, the severity of the condition, the route of administration, and the particular compound employed. Thus, the dosage regimen may vary widely, but can be determined routinely using standard methods.

In general, recombinant viruses may be administered in compositions in a dosage amount of about 10⁴ to about 10⁹ pfu per inoculation; often about 10⁴ pfu to about 10⁶ pfu, or as shown in the Examples, 10⁷ to 10³ pfu. Higher dosages such as about 10⁴ pfu to about 10¹⁰ pfu, e.g., about 10⁵ pfu to about 10⁹ pfu, or about 10⁶ pfu to about 10⁸ pfu, or about 10⁷ pfu can also be employed Another measure commonly used is cell culture infective dose (CCID₅₀); suitable CCID₅₀ ranges for administration include about 10¹, about 10², about 10³, about 10⁴, about 10⁵, about 10⁶, about 10⁷, about 10⁸, about 10⁹, about 10¹⁰ CCID₅₀. Ordinarily, suitable dosage amounts of plasmid or naked DNA are about 1 µg to about 100 mg, about 1 mg, about 2 mg, but lower levels such as 0.1 to 1 mg or 1-10 µg may be employed. For polypeptide compositions (e.g., AIDSVAX compositions), a suitable amount may be 1-1000 µg. Without limiting the possible subranges within that dosage range, Particular embodiments may employ 5, 10, 20, 50, 100, 150, 200, 250, 300, 350, 400, 450, 500, 550, 600, 650, 700, 750, 800, 850, 900, 950, and 1000 µg. A typical exemplary dosage of polypeptide may be, for example, about 50-250 µg, about 250-500 µg, 500-750 µg, or about 1000 µg of polypeptide. Low dose administration may typically utilize a dose of about 100 µg or less. High dose administration may typically utilize a dose of 300 µg or more. In referring to the amount of polypeptide in a dose, it is to be understood that the amount may refer to the amount of a single polypeptide or, where multiple polypeptides are administered, to the total amount of all polypeptides (e.g., 300 µg each of two polypeptides for a total administration of 600 µg). The AIDSVAX™ compositions described herein are typically but not necessarily administered in a total dosage of 200 µg or 600 µg (e.g., recombinant MN and GNE8 gp120, or recombinant MN and A244 gp120). "Dosage" may refer to that administered in a single or multiple doses, including the total of all doses administered. Actual dosages of such compositions can be readily determined by one of ordinary skill in the field of vaccine technology.

The pharmaceutical composition may be administered orally, parentally, by inhalation spray, rectally, intranodally, or topically in dosage unit formulations containing conventional pharmaceutically acceptable carriers, adjuvants, and vehicles. The term "pharmaceutically acceptable carrier" or "physiologically acceptable carrier" as used herein refers to one or more formulation materials suitable for accomplishing or enhancing the delivery of a nucleic acid, polypeptide, or peptide as a pharmaceutical composition. A "pharmaceutical composition" is a composition comprising a therapeutically effective amount of a nucleic acid or polypeptide. The terms effective amount" and "therapeutically effective amount" each refer to the amount of a nucleic acid or polypeptide used to observe the desired therapeutic effect (e.g., induce or enhance and immune response).

Injectable preparations, such as sterile injectable aqueous or oleaginous suspensions, may be formulated according to known methods using suitable dispersing or wetting agents and suspending agents. The injectable preparation may also be a sterile injectable solution or suspension in a non-toxic parenterally acceptable diluent or solvent. Suitable vehicles and solvents that may be employed are water, Ringer's solution, and isotonic sodium chloride solution, among others. For instance, a viral vector such as a poxvirus may be prepared in 0.4% NaCl or a Tris-HCl buffer, with or without a suitable stabilizer such as lactoglutamate, and with or without freeze drying medium. In addition, sterile, fixed oils are conventionally employed as a solvent or suspending medium. For this purpose, any bland fixed oil may be employed, including synthetic mono- or diglycerides. In addition, fatty acids such as oleic acid find use in the preparation of injectables.

Pharmaceutical compositions may take any of several forms and may be administered by any of several routes. The compositions are administered via a parenteral route (e.g., intradermal, intramuscular, subcutaneous, skin scarification) to induce an immune response in the host. Alternatively, the composition may be administered directly into a tissue or organ such as a lymph node (e.g., intranodal) or tumor mass (e.g., intratumoral). Preferred embodiments of administratable compositions include, for example, nucleic acids, viral particles, or polypeptides in liquid preparations such as suspensions, syrups, or elixirs. Preferred injectable preparations include, for example, nucleic acids or polypeptides suitable for parental, subcutaneous, intradermal, intramuscular or intravenous administration such as sterile suspensions or emulsions. For example, a naked DNA molecule and / or recombinant poxvirus may separately or together be in admixture with a suitable carrier, diluent, or excipient such as sterile water, physiological saline, glucose or the like. The composition may also be provided in lyophilized form for reconstituting, for instance, in isotonic aqueous, saline buffer. In addition, the compositions can be co-administered or sequentially administered with one another, other antiviral compounds, other anti-cancer compounds and/or compounds that reduce or alleviate ill effects of such agents.

As previously mentioned, while the compositions described herein may be administered as the sole active agent, they can also be used in combination with one or more other compositions or agents (i.e., other immunogens, co-stimulatory molecules, adjuvants). When administered as a combination, the individual components can be formulated as separate compositions administered at the same time or different times, or the components can be combined as a single composition. In one embodiment, a method of administering to a host a first form of an immunogen and subsequently administering a second form of the immunogen wherein the first and second forms are different, and wherein administration of the first form prior to administration of the second form enhances the immune response resulting from administration of the second form relative to administration of the second form alone, is provided. Also provided are compositions for administration to the host. For example, a two-part immunological composition where the first part of the composition comprises a first form of an immunogen and the second part comprises a second form of the immunogen, wherein the first and second parts are administered together or separately from one another such that administration of the first form enhances the immune response against the second form relative to administration of the second form alone, is provided. The immunogens, which may be the same or different, are preferably derived from the infectious agent or other source of immunogens. The multiple immunogen may be administered together or separately, as a single or multiple compositions, or in single or multiple recombinant vectors. For instance, a viral vector encoding an immunogen may be initially administered and followed by one or more subsequent administrations with a second form of the immunogen (e.g., a polypeptide). The different forms may differ in either or both of the form of delivery (e.g., viral vector, polypeptide) or in the immunogens represented by each form. It is preferred that the forms, however, induce or enhance the immune response against a particular target (e.g., HIV-1). For instance, as shown herein, a viral vector encoding a viral antigen (e.g., HIV gp120) may be administered to a human being. This may then be followed by administration of the viral vector along with a polypeptide representing the same or a similar viral antigen (e.g., HIV gp120). For prime-boost applications (e.g., ALVAC-HIV and AIDSVAX™), ALVAC-HIV is typically administered in a 10⁷ CCID₅₀ dosage (the "priming" dose), and then subsequently re-administered at the same or different dosage along with a sutiable dosage (e.g., 600 µg total, the "boosting" dose) of polypeptide (e.g., AIDSVAX™ B/B or B/E). ALVAC-HIV is a preparation of live attenuated, recombinant canarypox virus (ALVAC(1)) expressing gene products from the HIV-1 *env* (clade E in vCP1521 and clade B in vCP205), *gag* (clade B), and protease (clade B) coding sequences **(****Fig. 2****).** Exemplary, non-limiting prime-boost combinations may include ALVAC-HIV (vCP205) and AIDSVAX™ B/B or ALVAC-HIV (vCP1521) and AIDSVAX™ B/E, as the HIV clades from which the gp120 immunogen is derived in those combinations are the same. Typically, both the priming and boosting doses are administered via the same route (e.g., intramuscular, intradermal) but the routes of administration may also be different. Typically, the priming and boosting doses are administered to different parts of the body, but the doses may also be administered to the same part of the body. "Along with" may mean that the two forms are administered as separate compositions, as part of a single composition, at separate sites of the body, or at the same site of the body, depending on the particular protocol. Variations of such exemplary dosing regimens may be made by those of skill in the art.

A kit comprising a composition of the present invention is also provided. The kit can include a separate container containing a suitable carrier, diluent or excipient. The kit may also include additional components for simultaneous or sequential-administration. In one embodiment, such a kit may include a first form of an immunogen and a second form of the immunogen. Additionally, the kit can include instructions for mixing or combining ingredients and/or administration. A kit may provide reagents for performing screening assays, such as one or more PCR primers, hybridization probes, and / or biochips, for example.

A better understanding of the present invention and of its many advantages will be had from the following examples, given by way of illustration.

### EXAMPLES

### Example 1

### Immunological Compositions

### A. First composition: viral vector

ALVAC-HIV is a preparation of live attenuated, recombinant canarypox virus (ALVAC(1)) expressing gene products from the HIV-1 *env* (clade E in vCP1521 and clade B in vCP205), transmembrane anchoring portion of gp41 (clade B:LAI), *gag* (clade B:LAI), and protease (clade B:LAI) coding sequences and cultured in chick embryo fibroblast cells. These vectors were generated by co-insertion of genes encoding HIV-1 gene products into the ALVAC(1) genome at the C6 insertion site using standard techniques **(****Fig. 2A****).** The HIV-1 sequences contained within ALVAC-HIV (vCP1521) are shown in **Fig. 2B** and SEQ ID NOS.:5 and 6. These sequences include: 1) the region of the *env* gene encoding the extracellular envelope gp120 moiety of TH023 strain of HIV-1 linked to the sequences encoding the HIV-1 transmembrane anchor sequence of gp41 (28 amino acids), under the control of the vaccinia virus H6 promoter; and, 2) the *gag* gene encoding the entire Gag protein, and a portion of the *pol* sequences of LAI strain of HIV-1 sufficient to encode the protease function, under the control of the same vaccinia virus promoter I3L.

ALVAC-HIV was produced by inoculation of the ALVAC-HIV working seed lot in primary chick embryo fibroblasts and cultivation in roller bottles. After viral amplification, the infected cells were harvested and disrupted by sonication and cell debris removed by centrifugation. An equal volume of stabilizer (lactoglutamate) was blended with the supernatant and the suspension filtered through a 4.5 µm membrane. The clarified suspension was filled into vials and stored at ≤-35°C. At this step, the biological substance is the clarified harvest. End stage manufacturing of the vaccine entails blending of the clarified harvest with the freeze drying medium under sterile conditions. This blend (final bulk product) was prepared and then filled and freeze dried.

Immunoprecipitation analyses were performed using radiolabelled lysates derived from uninfected CEF cells or cells infected with either ALVAC(1) parental virus or ALVAC-HIV. Immunoprecipitation was performed using human serum derived from HIV-seropositive individuals (anti-HIV). Results with anti-HIV demonstrated expression of gp120, the 55 kDa precursor Gag polypeptide, and intermediate and completely processed forms of Gag including the major capsid protein, p24 in ALVAC-HIV infected CEF cells but not from cells infected with ALVAC parental virus.

Regarding ALVAC-HIV (vCP1521), FACS (Fluorescent Activated Cell Sorter) scan analyses with human anti-HIV antibody demonstrated expression of gp120 on the surface of infected HeLa, but not the parental virus. PCR amplification of the inserted sequences and those of ALVAC was performed. DNA analysis was performed by agarose gel electrophoresis followed by ethidium bromide staining to confirm the identity of the amplified fragments according to their molecular size. Restriction analysis was performed on viral DNA derived from ALVAC-HIV (vCP1521) infected cells to conform proper insertion of the gp120TM and gag expressing cassettes. The nucleotide sequence of inserted genes was confirmed by sequencing on the Working Seed Lot (passage 6).

ALVAC-HIV (vCP1521) genetic stability was confirmed by immunoplaque assay after several passages on CLEF. Immunoplaque analysis consisted of detecting Env and Gag expression in viral plaques by monoclonal antibodies. Analysis was performed after 7 passages (i.e. at the production lot level) and after 10 passages (i.e. 3 passages beyond the production lot level).

ALVAC-HIV (vCP1521) was formulated as a lyophilized vaccine for injection and reconstituted with 1.0 mL of sterile sodium chloride solution (NaCl 0.4%) for a single dose. The composition of the vaccine after reconstitution with 1.0 mL NaCl 0.4% included ALVAC-HIV (vCP1521): ≥10^{6.0} CCID₅₀ and excipients (TRIS-HCl buffer 10 mM, pH 9, 0.25 mL; stabilizer (lactoglutamate), 0.25 mL; freeze-drying medium, 0.50 mL; and NaCl, 4 mg). The appearance of the lyophilisate was homogeneous, white to beige; residual moisture was ≤ 3%; reconstitution time was ≤ 3 minutes; the appearance after reconstitution was a limpid to sightly opalescent solution, colorless with possible presence of particles or filaments; pH between 7.0 and 8.0; osmolality between 350 to 700 mOsmol/kg; BSA content of ≤50 ng/dose; bacterial endotoxins content of ≤10 IU/dose. The ALVAC-HIV (vGP1521) was stored at 2-8 °C without freezing and administered within 2 hours of reconstitution. Prior to reconstitution, the vial was allowed to come to room temperature. Each vial was reconstituted with the diluent supplied, 1.0 mL 0.4% NaCl for administration by slow injection into the vial containing the lyophilized ALVAC-HIV (e.g., using a 25 gauge, 5/8- inch needle). The vial was allowed to sit for approximately three minutes, and then gently swirled. The viral was then inserted and the contents withdrawn into a syringe.

### B. Second composition: polypeptide

Recombinant gp120 is an envelope glycoprotein with an apparent molecular mass of about 120,000 dalions. Approximately 50% of the molecular mass is accounted for by extensive glycosylation of the protein. AIDSVAX™ vaccines are highly purified mixtures of gp120 proteins from HIV-1 produced by recombinant DNA procedures using Chinese hamsters ovary (CHO) cell expression. Molecular epidemiologic analyses of virus circulating in the US has documented polymorphisms occur at the major neutralizing epitopes of gp120. Analysis of breakthrough infections in Phase I and Phase II trials of MN rgp120/HIV-1 revealed that most contained amino acid substitutions that differed from MN rgp120/HIV-1 at epitopes important for virus neutralization (specifically at the V2, V3 and C4 domains). After examining a variety of US strains, GNE8 was selected because amino acid sequences at sites know to be target of neutralizing antibodies differed from MN and possessed common polymorphisms that complemented MN at major neutralizing epitopes. Thus, an exemplary polypeptide composition is AIDSVAX™ B/B (VaxGen), which contains a bivalent polypeptide vaccine containing the HIV-1 type B epitopes MN recombinant glycoprotein (rgp)120 (amino acids 12-485 of MN gp120) and GNE8 rpg120 (amino acids 12-477 of GNE8 gp120) at a one-to-one ratio. Another exemplary polypeptide composition for use where subtypes B and E are prevelant (e.g., Thailand) is AIDSVAX™ B/E, which contains the subtype B antigen MN rgp120 (as described above) and the subtype E antigen A244 (CM244) recombinant glycoprotein 120 (amino acids 12.484 of A244 gp120) at a one-to-one ratio. The subtype E antigen is derived from the A244 (CM244) strain of HIV-1, which is isolated from Chiang Mai in Northern Thailand and represents about 75% of the incident infections in intravenous drug users (IVDUs) in Bangkok. A244 rgp120/HIV-1 is derived from a primary, macrophage or NSI viral type and, like GNE8 rgp120/HIV-1, requires the chemokine receptor CCR5 to bind with CD4 cells.

In both AIDSVAX™ B/B and AIDSVAX™ B/E, gp120 of the MN, GNE8, or A244 strains are each expressed as an amino-terminal fusion protein with a 27 amino acids of the herpes simplex virus type I gD protein. The gD sequence facilitates gp120 expression and provides an epitope that can be used in a generic immunoaffinity purification process. The amino acid residues equivalent to the mature, native gp120 for each particular HIV-1 isolate utilized are: 12 to 485 for the MN isolate, 12 to 477 for the GNE8 isolate, and 12 to 484 for the A244 isolate.

The recombinant gp120 polypeptides are produced in genetically modified CHO cell line. The CHO cells secrete the rgp120/HIV-1 molecule into the culture medium, and the protein is purified by a generic purification process for gp120 that includes immunoaffinity chromatography. AIDSVAX™ bivalent vaccines are supplied as a sterile suspension in single-use glass vials. Each vial has a nominal content of 1 mL (300 µ/mL) of each rgp120/HIV-1 protein adsorbed onto a total of 0.6 mg aluminum hydroxide gel adjuvant.

### Example 2

### Clinical Trial Design and Results

A prime-boost immunization protocol, using as the "prime" composition ALVAC-HIV (vCP1521) and the "boost" composition AIDSVAX^{®} B/E was tested in a formal phase III clinical trial. The trial described herein was a community-based, randomized (vaccine: placebo = 1:1), multicenter, double blind, placebo-controlled clinical trial conducted in Thailand. The primary objective of the study was to determine if vaccination with ALVAC-HIV (vCP1521) and AIDSVAX^{®} B/E could prevent HIV infection in healthy Thai adults. Thus, infection rates, as well as plasma viral load and CD4⁺ T cell counts in volunteers developing HIV infection during the trial, were assessed. The statistical assumptions of the study required that 16,000 persons enroll into the study. Intramuscular vaccinations (e.g., deltoid muscle) for each individual occurred during a 24-week period (0, 4, 12, 24 weeks). Following vaccination, the volunteers were tested for the presence of HIV in their plasma every 6 months for 3 years.

The "first composition" (ALVAC-HIV), as described in Example 1A above was initially administered to patients as the priming dose at months 0, 1, 3 and 6 (weeks 0, 4, 12 and 24). The "second composition" (AIDSVAX^{®} B/E) was later administered at months 3 and 6 (weeks 12 and 24 as for ALVAC-HIV) as the boosting dose. ALVAC-HIV doses included approximately 10⁷ CCID₅₀, AIDSVAX^{®} B/E doses included approximately 600 µg of polypeptide (300 µg of each of B and E). There was a 3 year follow-up with vaccine volunteers (Rerks-Ngram, 2009, NEJM 361:2209). The trial design is summarized in **Table 2,** as shown below:

**Table 2**

| ***RV144 Trial Design*** | | | | | |
|---|---|---|---|---|---|
| | | **Weeks** | | | |
| **Group** | **Number** | **0** | **4** | **12** | **24** |
| **I** | **8,000** | **ALVAC Placebo** | **ALVAC Placebo** | **ALVAC Placebo + AIDSVAX Placebo** | **ALVAC Placebo + AIDSVAX Placebo** |
| **II** | **8,000** | **ALVAC-HIV** | **ALVAC-HIV** | **ALVAC-HIV + AIDSVAX^{®} B/E** | **ALVAC-HIV + AIDSVAX^{®} B/E** |

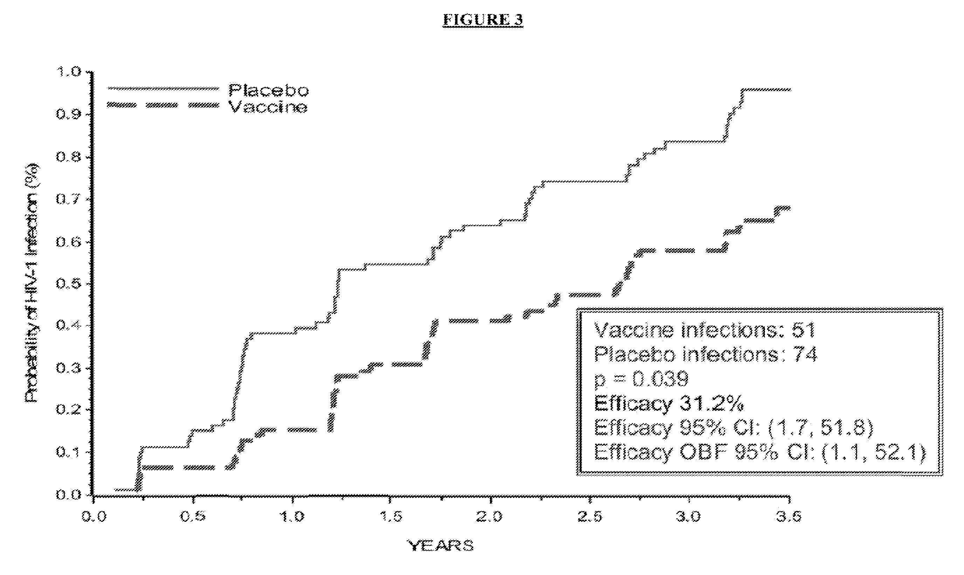

As shown in **Fig. 3****,** the two-part composition was used in a prime-boost format to successfully vaccinate human beings with an efficacy of 31,2% (e.g., about one-third of the population). The population to whom a placebo was administered exhibited 74 HIV infections over the testing period, while those administered the ALVAC-HIV / AIDSVAX^{®} B/E two-part composition ("vaccine") exhibited 51 HIV infections over the testing period. The difference in the number of HIV infections between the two groups was statistically significant (p=0.039). In patients that were infected by HIV after vaccination, neither the setpoint viral load nor mean CD4+ T cell counts were significantly different from placebo at 30 months post-vaccination. This is the first vaccine shown to reduce the risk of HIV infection in human beings compared to a placebo. The observed vaccine efficacy was 31.2% (p = 0.0385, O'Brien-Fleming adjusted 95% CI 1.1, 52.1) using the Cox Proportional Hazard method. Thus, this is the first demonstration of a safe, efficacious vaccine that is capable of protecting human beings from infection by HIV.

It is to be understood that any reference to a particular range includes all individual values and sub-ranges within that range as if each were individually listed herein. All references cited within this application are incorporated by reference in their entirety. While the present invention has been described in terms of the preferred embodiments, it is understood that variations and modifications will occur to those skilled in the art. Therefore, it is intended that the appended claims cover all such equivalent variations that come within the scope of the invention as claimed.

### References

Burton, et al. Science, 303:316 (2004)
de Bruyn, et al. Vaccine. 22:704-13(2004)
Esposito, et al. (eds): Arch. Virol. Supp.2. NY, Springer-Verlag, pp 91-102 (1991)
Fang, et al. J. Infect. Dis. 180 (4): 1122-32 (1999)
Ferrari, et al. Proc. Natl. Acad. Sci. USA 94:1396-401 (1997)
Klausner, et al. Science, 300:2036-39 (2003)
Letters to the Editor. Science, 305:177-180 (2004)
McCarthy M. Lancet. Nov 22;362(9397):1728 (2003)
McNeil, et al. Science, 303:961 (2004)
Meeting Summary: AIDS Vaccine Trials, Considerations for Phase III Trial Design and Endpoints. National Institutes of Health, NIAID, November 16, 2001. Retrieved from the World Wide Web on August 3, 2004 at http://www.niaid.nih.gov/vrc.
NIAID Phase III HIV vaccine trial to determine correlates of protection will not proceed. NIAID News, February 25, 2002. Retrieved from the World Wide Web on August 9, 2004 at http://www2.niaid.nih.gov/newsroom/releases/phase3hiv.htm.
Nitayaphan, et al. J. Inf. Dis. 190:702-6 (2004)
Rerks-Ngarm et al NEJM, 361:2209 (2009)
Tangcharoensathien, et al. Health Policy. 57:111-139 (2001)
Tartaglia, et al. (eds): AIDS Research Reviews, Vol.3. New York, Marcel Dekker, pp 361-378(1993).
Tovanabutra S, et al. AIDS Vaccine 2003, New York, NY; Abstract number 463.
Trinvuthipong, D. Science, 303:954-5 (2004)

### Paragraphs of the Invention:

1. A method for protectively immunizing a human being against human immunodeficiency virus (HIV) by administering to the human being at least one dose of a first composition comprising a viral vector encoding an HIV polypeptide or fragment or derivative thereof and subsequently administering to the human being at least one dose of a second composition comprising the HIV polypeptide or fragment or derivative thereof, wherein a protective immune response directed against HIV is induced.
2. A method for protectively immunizing a human being against human immunodeficiency virus (HIV) by administering to the human being a vaccine consisting essentially of:
   a first composition and a second composition,
   the first composition consisting essentially of a live, attenuated viral vector encoding at least one HIV gp120 or fragment or derivative thereof and, optionally, at least one additional HIV polypeptide or fragment or derivative thereof,
   the second composition consisting essentially of at least one HIV gp120 polypeptide or
   fragment or derivative thereof and, optionally, at least one additional HIV polypeptide
   or fragment or derivative thereof;
   the method comprising the steps of administering the first composition to the human being and subsequently administering to the human being at least one composition or combination of compositions selected from the group consisting of:
   the second composition alone;
   the first and second compositions, optionally separately or together as a single dose;
   at least one additional dose of the first composition followed by at least one dose of the second composition;
   the second composition followed by at least one additional dose of the first composition, optionally followed by at least one additional dose of the first and / or second composition;
   wherein a protective immune response against HIV is induced in the human being.
3. The method of paragraph 1 or 2 wherein at least one of the compositions comprises an amino acid sequence corresponding to that of a herpes simplex virus (HSV).
4. The method of paragraph 3 wherein the HSV amino acid sequence comprises SEQ ID NO.: 7.
5. The method of paragraph 1 or 2 wherein one of the compositions comprises SEQ ID NO. 7.
6. The method of paragraph 1 or 2, further comprising administering the vaccine is administered to a population of human beings and at least about one-third of that population is protected from infection by HIV.
7. The method of any one of paragraphs 1-3, wherein the first composition is administered repeatedly prior to at least one administration of the second composition, with the time between administrations is of sufficient length to allow for the development of an immune response within the human being.
8. The method of paragraph 4, wherein the second composition is co-administered with the first composition.
9. The method of any one of paragraphs 1-5, wherein administration of both the first and second compositions is via a route selected from the group consisting of mucosal, intradermal, intramuscular, subcutaneous, via skin scarification, intranodal, or intratumoral.
10. The method of any one of paragraphs 1-6, wherein administration is at separate sites in the human being.
11. The method of any one of paragraphs 1-7 wherein the amount of viral vector administered in each dose is the equivalent of about 10⁷ CCID₅₀ and the total amount of polypeptide administered in each dose is about 600 µg.
12. The method of any one of paragraphs 1-11 wherein the viral vector is selected from the group consisting of retrovirus, adenovirus, adeno-associated virus (AAV), alphavirus, herpes virus, and poxvirus.
13. The method of paragraph 12 wherein the viral vector is a poxvirus.
14. The method of paragraph 13 wherein the poxvirus vector is selected from the group consisting of vaccinia, NYVAC, Modified Virus Ankara (MVA), avipox, canarypox, ALVAC, ALVAC(2), fowlpox, and TROVAC.
15. The method of paragraph 14 wherein the viral vector is a poxvirus selected from the group consisting of NYVAC, ALVAC, and ALVAC(2).
16. The method of any one of paragraphs 1-15 wherein the HIV polypeptide or HIV gp120 is derived from an HIV virus selected from the group consisting of HIV-1, HIV-2, and HIV-3, wherein the first and second composition contain the same or different HIV polypeptides and / or gp120.
17. The method of paragraph 16 wherein the HIV-1 is HIV-1 subtype A1, HIV-1 subtype A2, HIV-1 subtype A3, HIV-1 subtype A4, HIV-1 subtype B, HIV-1 subtype C, HIV-1 subtype D, HIV-1 subtype E, HIV-1 subtype F1, HIV-1 subtype F2, HIV-1 subtype G, HIV-1 subtype H, HIV-1 subtype J and HIV-1 subtype K.
18. The method of paragraph 16 wherein the HIV-2 is selected from the group consisting of HIV-2 subtype A, HIV-2 subtype B, HIV-2 subtype C, HIV-2 subtype D, and HIV-2 subtype E.
19. The method of any one of paragraphs 1-18 further comprising administering a composition comprising at least one additional HIV immunogen selected from the group consisting of gag, pol, nef, a variant thereof, and a derivative thereof.
20. The method of any one of paragraphs 1-19 wherein the first or second composition additionally contain at least one additional HIV immunogen selected from the group consisting of gag, the protease component encoded by *pol,* nef, a variant thereof, and a derivative thereof.
21. The method of any one of paragraphs 1-20 wherein the viral vector encodes at least one polypeptide selected from the group consisting of HIV gp120 MN 12-485, HIV gp120 A244 12-484, and HIV gp120 GNE8 12-477.
22. The method of any one of paragraphs 1-20 wherein the viral vector encodes at least HIV gp120 MN 12-485 and HIV gp120 GNE8 12-477, or at least HIV gp120 MN 12-485 and HIV gp120 A244 12-484.
23. The method of any one of paragraphs 1-22 wherein the viral vector is ALVAC-HIV (vCP1521).
24. The method of any one of paragraphs 1-23 wherein the viral vector comprises the nucleic acid sequence of SEQ ID NO. 5.
25. The method of any one of paragraphs 1-24 wherein the second composition is AIDSVAX^{®} B/B or AIDSVAX^{®} B/E.
26. The method of any one of paragraphs 1-25 wherein the viral vector is ALVAC-HIV (vCP1521) and the second composition is AIDSVAX^{®} B/E.

## Claims

1. A vaccine comprising a first composition and a second composition for use in protectively immunizing a human being against human immunodeficiency virus (HIV) wherein at least one dose of the first composition is administered to the human and at least one dose of the second composition is subsequently administered to the patient, said first composition comprising a viral vector encoding an HIV polypeptide or fragment or derivative thereof and the second composition comprises the HIV polypeptide or fragment or derivative thereof.

2. A vaccine for use as claimed in claim 1 wherein the first composition comprises an attenuated viral vector encoding at least one HIV gp120 polypeptide or fragment or derivative thereof and the second composition comprises at least one HIV gp120 polypeptide or fragment thereof.

3. A vaccine for use as claimed in either of claims 1 or 2 wherein a first composition is administered repeatedly and the second composition is administered repeatedly.

4. A vaccine for use as claimed in claim 3 wherein the first composition is administered repeatedly before the second composition is administered.

5. A vaccine for use as claimed in claims 3 or 4 wherein at least one dose of the first composition and at least one dose of the second composition are administered together.

6. A vaccine for use as claimed in claims 1 to 5 wherein the second composition is adjuvanted.

7. A vaccine for use as claimed in claim 6 wherein the adjuvant is aluminium hydroxide.

8. A vaccine for use as claimed in any of claims 1 to 7 wherein the first composition comprises 10⁷ CCID₅₀.

9. A vaccine for use as claimed in any of claims 1 to 8 wherein the second composition comprises 600µg of polypeptide.

10. A vaccine for use as claimed in any of claims 1 to 7 wherein the viral vector is a poxvirus.

11. A vaccine for use as claimed in claim 10 wherein the poxvirus is ALVAC.

12. A vaccine for use as claimed in claim 11 wherein the ALVAC poxvirus expresses HIV-1 *env* from clade B or clade E.

13. A vaccine for use as claimed in claim 11 wherein the viral vector is vCP1521 or vCP205.

14. A vaccine for use as claimed in any of claims 1 to 12 wherein the second composition comprises AIDSVAX B/B or AIDSVAX B/E.

15. A vaccine for use as claimed in claims 12 or 13 wherein the second composition comprises HIV-1 type B epitopes MN recombinant glycoprotein (amino acids 12-485 of MN gp120) and GNE8 rpg120 (amino acids 12-477 of GNE8 gp120).

16. A vaccine for use as claimed in claims 12 or 13 wherein the second composition comprises subtype B antigen MN rgp120 and subtype E antigen A244 (CM244) recombinant glycoprotein 120 (amino acids 12-484 of A244 gp120).
